(19) Europäisches Patentamt
European Patent Office
Office européen des brevets

(11) EP 3 639 829 B1

(12) **EUROPEAN PATENT SPECIFICATION**

(45) Date of publication and mention of the grant of the patent:
**09.02.2022 Bulletin 2022/06**

(21) Application number: **18827765.1**

(22) Date of filing: **04.07.2018**

(51) International Patent Classification (IPC):
***A61K 31/7048*** (2006.01) ***A61P 35/00*** (2006.01)
***A61P 35/02*** (2006.01) ***A61K 45/06*** (2006.01)

(52) Cooperative Patent Classification (CPC):
**A61K 31/7048; A61K 9/0019; A61K 9/0053; A61K 9/0095; A61K 9/145; A61K 9/1623; A61K 9/19; A61K 9/2027; A61K 9/2054; A61K 9/4866; A61K 45/06; A61P 31/00; A61P 31/04; A61P 31/10**

(86) International application number:
**PCT/CN2018/094504**

(87) International publication number:
**WO 2019/007368 (10.01.2019 Gazette 2019/02)**

(54) **USE OF ISOVALERYLSPIRAMYCIN I OR III IN PREPARATION OF DRUG FOR TREATING AND/OR PREVENTING TUMOUR, AND DRUG**

VERWENDUNG VON ISOVALERYLSPIRAMYCIN I ODER III ZUR HERSTELLUNG EINES ARZNEIMITTELS ZUR BEHANDLUNG UND/ODER VORBEUGUNG VON TUMOREN UND ARZNEIMITTEL

UTILISATION DE L'ISOVALÉRYLSPIRAMYCINE I OU III DANS LA PRÉPARATION D'UN MÉDICAMENT POUR LE TRAITEMENT ET/OU LA PRÉVENTION D'UNE TUMEUR, ET UN MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**04.07.2017 CN 201710537958**
**27.12.2017 CN 201711497546**
**27.12.2017 CN 201711450300**
**27.12.2017 CN 201711450396**

(43) Date of publication of application:
**22.04.2020 Bulletin 2020/17**

(73) Proprietor: **Shenyang Fuyang Pharmaceutical Technology Co., Ltd.**
**Shenyang, Liaoning 110164 (CN)**

(72) Inventors:
- **JIANG, Enhong**
**Shenyang**
**Liaoning 110164 (CN)**
- **HE, Weinqing**
**Shenyang**
**Liaoning 110164 (CN)**
- **DAI, Jianlu**
**Shenyang**
**Liaoning 110164 (CN)**
- **JIANG, Yang**
**Shenyang**
**Liaoning 110164 (CN)**
- **JIANG, Xulein**
**Shenyang**
**Liaoning 110164 (CN)**
- **ZHAO, Xiaofeng**
**Shenyang**
**Liaoning 110164 (CN)**

(74) Representative: **Murgitroyd & Company**
**Murgitroyd House**
**165-169 Scotland Street**
**Glasgow G5 8PL (GB)**

(56) References cited:
**EP-A1- 2 578 596** **EP-A1- 3 607 952**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 639 829 B1

WO-A1-2007/144876 CN-A- 101 568 343
CN-A- 101 785 778 CN-A- 102 229 634

## Description

### TECHNICAL FIELD

The present disclosure belongs to the technical field of pharmaceuticals, and specifically relates to isovaleryl spiramycin I or III for use in the treatment or prevention of a tumor.

### BACKGROUND

Tumor is a common and frequently-occurring disease, and refers to the neoformation or neoplasm formed by clonal abnormal proliferation and differentiation caused by genetic mutation and loss of normal regulation of growth and differentiation of the histocyte of the organism under the long-term action of tumorigenic factors in vivo and in vitro. Tumors are classified into benign tumors and malignant tumors. The malignant tumors are further divided into three types: carcinomas derived from epithelial tissues, sarcomas derived from mesenchymal tissues, and carcinosarcomas. The term "cancer" is generally used to refer to all malignant tumors.

The malignant tumors are one of the major malignant diseases threatening human health and the first cause of death of the world's population. According to the latest statistics, in 2007, about 7.9 million people in the world died of various cancers, accounting for 13% of all deaths, and more than 12 million cancer cases were diagnosed, wherein 72% or more of tumor patients and deaths have occurred in underdeveloped countries, and it is rising continuously. In 2015, 9 million people in the world died of tumors, and it is expected that more than 12 million people will die of tumors in 2030. At present, the annual number of cancer cases in China is about 2.8 million, and the number of cancer deaths is more than 400,000, ranking first among all kinds of diseases in China, and showing a rising trend. With the speeding up of the pace of social life, the increasing pressure of competition, and the changes of human lifestyle and environment, tumor cases and deaths are rising year by year, and tumors have become the common diseases and the high incidence in modern society, not only seriously affecting the patients' life quality, but also bringing heavy economic and mental burden to the patients' families and the society. And tumors are also important social problems in the world, the treatment and prevention of cancer have always been one of the most pressing issues in the world. At present, chemotherapy is a main means of fighting against tumors. Although the chemotherapy has a better curative effect, the chemotherapy often causes side effects such as myelosuppression and low immune functions, making it difficult for patients to adhere to treatment. And drug resistance in the treatment process of chemotherapy has become one of the difficult problems in the current clinical treatment. In recent years, the global market of anti-tumor drugs has been growing rapidly. According to the statistics of the US FDA, the total sales of anti-cancer drugs in the world increased from 24 billion US dollars in 2004 to 39.6 billion US dollars in 2007. Although new anti-tumor drugs come out every year in the world, so far, there is still no effective means for humans to fight against cancer. At the same time, new types of cancer are constantly discovered, and the emergence and enhancement of tumor resistance/drug resistance makes the need to find new effective anti-cancer drugs more and more urgent.

European patent application number EP3607952A1 describes the use of carrimycin and pharmaceutically acceptable salts thereof for the preparation of a medicament for the treatment of tumors. PCT patent publication number WO2007/144876A1 describes the use of a macrolide antibiotic for the manufacture of a medicament for the treatment or prevention of a particular cancer. European patent application number EP2578596A1 describes the preparation, use and pharmaceutical compositions or levocarrimycin. Chinese patent application number CN101785778A describes the separation of an antibiotic comprising isovaleryl-spiramycin I and the application of the antibiotic in resistant infectious diseases.

Carrimycin is a new type of antibiotic with the 4"- isovaleryl spiramycin as a main component, and carrimycin is formed by cloning the 4"-o-acyl-transferase of the carbomycin producing strain into a spiramycin producing strain by a transgenic technology, directionally acylating spiramycin 4"-OH, and adding an isovaleryl side chain at the 4"-position.

Carrimycin is composed of a variety of spiramycin derivatives with the main active component isovaleryl spiramycin (I+II+III) having a total content of no less than 60%, and is a pharmaceutically acceptable pharmaceutical composition. The central structure of the main component of the carrimycin is a 16-membered lactone ring, and the 16-membered lactone ring links one molecule of forosamine, one molecule of mycaminose and one molecule of mycarose. Its main component isovaleryl spiramycin I, II, III differs from the spiramycin structure in that the group connected to the 4'-position of mycarose is isovaleryl rather than hydroxyl. The drug is jointly declared by Tonglian Shengyang Group as the 1.1 type of new drug.

The chemical structure of the main component of carrimycin is shown as in a formula (I):

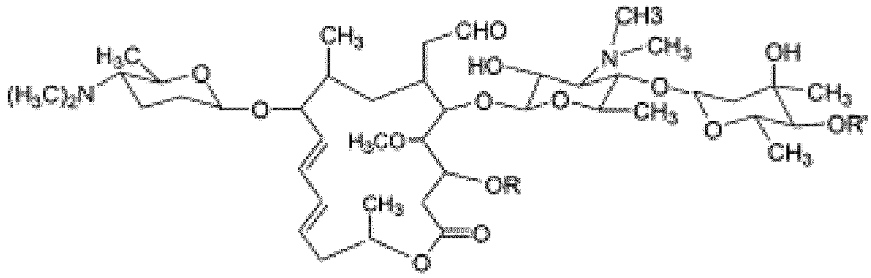

Formula (I)

Wherein, when R=H, R'=$COCH_2CH(CH_3)_2$, the main component is isovaleryl spiromycin I;
When R=$COCH_3$, R'=$COCH_2CH(CH_3)_2$, the main component is isovaleryl spiromycin II;
When R=$COCH_2CH_3$, R'=$COCH_2CH(CH_3)_2$, the main component is isovaleryl spiromycin III;

Carrimycin belongs to 16-membered macrolide antibiotics, has active groups such as a carboxyl group, an alkoxy group, an epoxy group, a ketone group and an aldehyde group, and a pair of conjugated C=C, and has a molecular weight of about 884 to 982. Carrimycin and macrolide antibiotics have many commonalities due to their similar chemical structures: they are easily soluble in most organic solvents such as esters, acetone, chloroform, alcohols, etc., slightly soluble in petroleum ether, and insoluble in water. Due to the presence of two dimethylamine groups in the molecular structure, carrimycin is alkalescence and easily soluble in an acidic aqueous solution. Carrimycin has a "negative solubility" property in which the solubility decreases with an increase temperature. Since isovaleryl spiramycin, the main component of carrimycin, has a longer carbon chain at the 4"-position and a poor hydrophilicity, the solubility of carrimycin in water is smaller than that of spiramycin and 4"-acetylspiramycin.

Carrimycin is a white amorphous powder with slight hygroscopicity, and specific rotation of about -80.8°, maximum ultraviolet absorption wavelength of 231-232 nm. Carrimycin contains weak fluorescent chromophores, presents a purple reaction producing a strong purple fluorescence in case of concentrated sulfuric acid or hydrochloric acid, and has a maximum absorbance at 231-232 nm.

The drug has good lipophilicity, strong tissue penetration ability, rapid oral absorption, long body maintenance time, and sustained post antibiotic effects. According to a relationship between pharmacodynamics and chemical conformation, after the 4"-position of the macrolide antibiotics acylation, macrolide antibiotics have improved lipophilicity and in vivo activity, and significantly improved in vivo antibacterial activity and clinical therapeutic effects, and the in vivo stability of antibiotics enhances with the growing of the carbon chain of the 4"-hydroxy ester, that is, isovaleryl spiramycin > butyryl spiramycin > propionyl spiramycin > acetyl spiramycin.

The preliminary in vitro and in vivo pharmacodynamic tests showed that the drug not only has good antibacterial activity against most $G^+$ bacteria, but also has certain effects on some $G^-$bacteria, and its technical indicators are obviously superior to those of azithromycin, erythromycin, acetyl spiramycin, and medemycin. It has the strongest antimicrobial activity especially against mycoplasma pneumoniae, also has certain antimicrobial activity against the erythromycin resistant bacteria, gonococcus, pneumococcus, staphylococcus aureus, bacillus pyocyaneus, bacillus influenzae, haemophilus influenzae, bacteroides fragilis, legionella, multi-line bacillus and clostridium perfringens, and a tiny cross resistance against staphylococcus aureus with clinical resistance to the erythromycin. Carrimycin will be primarily used to treat gram-positive infections, especially upper respiratory tract infections, and may be used for urinary tract infections.

In a recent study, the applicant found that through the evaluation of isovaleryl spiramycin I, II and/or III against the in vitro antiproliferative activity of human breast cancer cells MCF-7 and MDA-MB-231, human hepatoma cells HepG2 or murine hepatoma cells $H_{22}$, human non-small cell lung cancer cells A549, Lewis lung cancer cells, human large cell lung cancer cells H460 and H1299, human renal clear cell adenocarcinoma cell 786-O, human renal cell adenocarcinoma cell 769-P, human glioma cell U251, human glioblastoma cell A172, human tissue lymphoma cell U937, human cervical cancer cell HeLa, human prostate cancer cell PC3, human pancreatic cancer cell PANC-1, human esophageal cancer cell TE-1, human gastric adenocarcinoma cell SGC7901, human colon cancer cell HT-29, human promyelocytic leukemia cell HL-60, human thyroid cancer cell TPC-1, and human bladder cancer cell T-24, the samples showed good antiproliferative activity against the cells tested, indicating that isovaleryl spiramycin I, II and/or III is expected to be a new drug for treating tumors, thereby completing the present disclosure.

The invention is defined by the claims. Any information provided herein and falling outside the scope of the invention is provided for information purposes only.

The technical problem to be solved by the present disclosure is to overcome the defects of the prior art and provide use of isovaleryl spiramycin I or III in manufacturing medicament for treating and/or preventing tumor.

To solve the above technical problem, the present disclosure adopts the following technical solution:
The present disclosure firstly provides use of isovaleryl spiramycin I or III in manufacturing medicament for the treating and/or preventing tumor.

The tumor includes solid tumor and non-solid tumor.

Further, the solid tumor includes benign solid tumor and malignant solid tumor; the non-solid tumor is lymphoma or leukemia.

Further, the malignant solid tumor is breast cancer, liver cancer, lung cancer, renal cancer, brain tumor, cervical cancer, prostate cancer, lymphoma, pancreatic cancer, esophageal cancer, gastric cancer, colon cancer, thyroid cancer, bladder cancer, or malignant skin tumor;

Preferably, the brain tumor is glioma or meningioma, and the gastric cancer is gastric adenocarcinoma.

The present disclosure shows by experiments that isovaleryl spiramycin I, II and/or III show good antiproliferative activity on human breast cancer cells MCF-7 and MDA-MB-231, human hepatoma cells HepG2, human non-small cell lung cancer cells A549, human large cell lung cancer cells H460 and H1299, human renal clear cell adenocarcinoma cell 786-O, human renal cell adenocarcinoma cell 769-P, human tissue lymphoma cell U937, human cervical cancer cell HeLa, human prostate cancer cell PC3, human glioma cell U251, human glioblastoma cell A172, human pancreatic cancer cell PANC-1, human colon cancer cell HT-29, human esophageal cancer cell TE-1, human gastric adenocarcinoma cell SGC7901, human promyelocytic leukemia cell HL-60, human thyroid cancer cell TPC-1, and human bladder cancer cell T-24, confirming that isovaleryl spiramycin I, II and/or III can be used for treating these tumors or cancer diseases.

Further, the medicament is in various formulations made of isovaleryl spiramycin I or III and pharmaceutically acceptable salts of isovaleryl spiramycin I or III and pharmaceutically acceptable adjuvants.

Further, the medicament is in various formulations made of isovaleryl spiramycin I or III and the pharmaceutically acceptable salts of isovaleryl spiramycin I or III and anti-tumor drugs and the pharmaceutically acceptable adjuvants.

In the present disclosure, the isovaleryl spiramycin I, or III and at least one of the anti-tumor drugs can be formulated into a compound preparation.

Further, when preparing the compound preparation, the dosage ratio of isovaleryl spiramycin I or III to a second active component is 1-99: 99-1, preferably 5-95:95-5, more preferably 10-90:90-10, further preferably 20-80:80-20.

Further, the medicament is a combination of a first agent and a second agent, the first agent contains isovaleryl spiramycin I, or III and the pharmaceutically acceptable salts of isovaleryl spiramycin I, or III, and the second agent contains an anti-tumor drug.

In the present disclosure, the first agent containing the active component isovaleryl spiramycin I, or III can be used together with the second agent containing one or more than one drug selected from a group containing a chemotherapy drug, a radiotherapy drug, a targeted therapy drug or an immunotherapeutic drug. When they are used in combination, the first agent and the second agent can be administered in no particular order, namely, the first agent may be used first, or the second agent may be used first, or both are used simultaneously.

When they are used in combination, the dosage ratio of the first agent to the second agent is 1-99: 99-1, preferably 5-95:95-5, more preferably 10-90:90-10, further preferably 20-80:80-20.

Further, the anti-tumor drug is a chemotherapy drug, a radiotherapy drug, a targeted therapy drug, and/or an immunotherapeutic drug.

The present disclosure also provides a medicament for treating and/or preventing tumor, the active component of the medicament includes isovaleryl spiramycin I, or III.

Further, the medicament also includes a second active component.

Further, the second active component includes one or more than one drug selected from a group containing a chemotherapy drug, a radiotherapy drug, a targeted therapy drug and an immunotherapeutic drug.

The present disclosure also provides a combination product for treating and/or preventing tumor, the combination product includes a first agent and a second agent, and the active component of the first agent is isovaleryl spiramycin I or III and the second agent includes one or more than one drug selected from a group containing a chemotherapy drug, a radiotherapy drug, a targeted therapy drug and an immunotherapeutic drug.

Further, the medicament or the first agent is a pharmaceutically acceptable formulation.

Further, a dose of isovaleryl spiramycin I, or III in the medicament or the first agent is in a range from 5 to 1,500 mg; preferably in a range from 50 to 1,000 mg; more preferably in a range from 100 to 400 mg.

In the present disclosure, isovaleryl spiramycin I can be separated and prepared according to the methods of the prior art, such as the isovaleryl spiramycin I can be separated and prepared according to a method of Example 1 in CN101785778A. Isovaleryl spiramycin II can be separated and prepared according to the methods of the prior art, such as the isovaleryl spiramycin II can be separated and prepared according to a method of Example 1 in CN101785779A. Isovaleryl spiramycin III can be separated and prepared according to the methods of the prior art, such as the isovaleryl spiramycin III can be separated and prepared according to a method of Example 1 in CN101773510A.

After adopting the above technical solution, the disclosure has the following beneficial effects compared with

the prior art:
The present disclosure shows that isovaleryl spiramycin I, or III have good anti-tumor effects, especially have good curative effects on tumors including breast cancer, liver cancer, lung cancer, lymphoma, cervical cancer, prostate cancer, colon cancer or leukemia. The present disclosure not only provides a theoretical basis for the application and clinical promotion of isovaleryl spiramycin I, or III in the preparation of drugs for treating and/or preventing tumor, but also has important economic and social benefits.

## DETAILED DESCRIPTION OF THE EMBODIMENTS

To make the objectives, technical solutions and advantages of the embodiments of the present disclosure clearer, a clear and complete description of the technical solutions in the present embodiments will be given below. The following embodiments are used to illustrate the present disclosure, but are not used to limit the protection scope of the present disclosure.

Embodiment 1 Isovaleryl spiramycin I tablets

Specification: 200mg/350mg
Tablet Core Prescription:

| | |
|---|---|
| Isovaleryl spiramycin I | 200g |
| Microcrystalline cellulose | 110g |
| Sodium carboxymethyl starch | 22g |
| Povidone $K_{30}$ (5%) | 15g |
| Magnesium stearate | 3g |
| Prepared into | 1,000 tablets |

Coating Liquid Prescription:

| | |
|---|---|
| Opadry II | 21g |
| Distilled water | appropriate amount |
| Prepared into | 105ml |

Preparation Process:

Preparation of the tablet core: the main drug and adjuvants respectively were made to pass through a 100-mesh sieve, and a prescription amount of isovaleryl spiramycin I, a prescription amount of microcrystalline cellulose and a 1/2 prescription amount of sodium carboxymethyl starch were uniformly mixed, and then a 5% povidone $K_{30}$ aqueous solution was added to make a soft material. A 18-mesh sieve was used for granulating, and the wet granules were dried under a ventilated condition at 60°C for 2 hours. After the wet granules were, a 18-mesh sieve was used for dispersing the granules, and then a 1/2 prescription amount of sodium carboxymethyl starch and prescription amount of magnesium stearate were added. And after the materials were uniformly mixed, the mixture was tabletted with a shallow concave stamping die having a diameter of 11 mm to obtain a tablet core containing drugs, wherein the tablet is 350 mg in weight and 6.5 kg in hardness.
Preparation of the coating liquid: the required Opadry II (white) was weighed, the required amount of water was added into a liquid preparation container, the Opadry II was added into the liquid preparation container in batch. After all the Opadry II was added, the stirring speed was reduced to make the spiral disappear, and then stirring was continued to be performed for 30min to obtain the coating liquid.
Preparation of thin film coated tablets: the tablet core was placed in a coating pan, the coating conditions were determined, and coating was carried out with the host speed of 20 r/min, the inlet air temperature of 40°C, the outlet air temperature of 30°C, the spray pressure of 0.02 Mpa, and the spray slurry flow rate of 1ml/min. And after a constant state was achieved, the coating was continuously to be sprayed for 1.5 hours until the surfaces of the tablets were smooth and uniform in color, wherein tablets which were in compliance with the inspection standard of film coatings were qualified. The coating gains about 5% in weight.

Embodiment 2 Isovaleryl spiramycin I tablets (calculated by 10,000 tablets)

Prescription:

| | |
|---|---|
| Isovaleryl spiramycin I raw powder | 1000g |
| Low-substituted hydroxypropyl cellulose (5%) | 92.5g |
| Sodium carboxymethyl starch (3%) | 55.5g |
| Magnesium stearate (1%) | 18.5g |
| Starch | Total weight- minus the weight of other raw and auxiliary materials |
| Total weight | 1,850g |

Preparation process: an appropriate amount of starch was weighed, diluted to a concentration of 15%, and heated to a paste to get a binder; the main material isovaleryl spiramycin I, and the adjuvants starch, low-substituted hydroxypropyl cellulose, sodium carboxymethyl starch, and magnesium stearate pass through a 100-mesh sieve, respectively, and the required main material and adjuvants were weighed according to the prescription amount. After the isovaleryl spiramycin I, starch and low-substituted hydroxypropyl cellulose were fully and uniformly mixed, the starch paste with a starch concentration of 15% was used to prepare the mixture into a soft material which was granulated by a 14-mesh sieve, and granules were dried at 50-60°C to control a water content to be 3-5%. A 14-mesh sieve was used for dispersing the granules, and then sodium carboxymethyl starch and magnesium stearate were added to be mixed, and the granule content was measured. The weight of the tablet was calculated according to the granule content, and the mixture was tabletted (with a Φ9 mm shallow concave punch), then the difference in the weight of the tablets was detected. After passing the test, the tablets were packaged.

Embodiment 3 Isovaleryl spiramycin I capsules (calculated by 10,000 granules)

Prescription:

| | |
|---|---|
| Isovaleryl spiramycin I raw powder | 1,000g |
| Starch | 1,080g minus the weight of isovaleryl spiramycin I raw powder |
| No.3 medicinal capsule | 1,000 granules |
| Liquid paraffin | 50ml |

Preparation process: the main material isovaleryl spiramycin I and the adjuvant medicinal starch were separately weighed according to the process formula amount, and then fully mixed in a mixer for 1.5-2 hours. The data obtained by sampling and content testing should be basically consistent with the theoretical data (the weight of each capsule was about 0.105g), and the qualified No. 3 medicinal capsule and the mixed raw materials to be loaded were filled in a filling device according to the operation requirements of an automatic capsule machine, and the filled capsules were subjected to a difference test (±10% or less, <0.3g) to see if the dissolution rate meets the requirements or not, the capsules that meet the requirements after being tested were put into a polishing machine to be polished for 15-20 minutes with the liquid paraffin added, and then were taken out to be tested by finished product packaging boxes.

Embodiment 4 Isovaleryl spiramycin I dry syrup (calculated according to 10,000 bags)

Prescription:

| | |
|---|---|
| Isovaleryl spiramycin I raw powder | 1,250g |
| Citric acid (0.5%) | 15g |
| Sucrose | total weight minus other raw and auxiliary materials |
| Total weight | about 500g |
| Pigment (curcumin) | about 1g |

Preparation process: the isovaleryl spiramycin I raw powder, citric acid and sucrose were respectively grinded into granules by a high-speed jet mill, and 85% of the granules pass through a 300-mesh sieve, 15% of the granules pass through a 180-mesh sieve. Then the fine powder after grinding was weighed according to the prescription amount

and fully mixed for 1-1.5 hours, the content was measured, the loading capacity was calculated (the theoretical loading capacity is 500mg per bag). Then the mixture was put into a bagging machine, aluminum foil paper was installed, and filling was carried out according to the operation requirements of a filling machine. The difference was allowed to be within ±5 %, and after the filling, the outer packaging was carried out after passing the inspection.

Embodiment 5 Isovaleryl spiramycin I granules (calculated according to 10,000 bags)

Prescription:

| | |
|---|---|
| Isovaleryl spiramycin I raw powder | 1,250g |
| Powdered sugar | 20,000g |
| Dextrin | 9,000g |
| 5% PV-P-$K_{30}$ | appropriate amount |

Preparation process: the isovaleryl spiramycin I raw powder, powdered sugar and dextrin were made to pass through a 120-mesh sieve, and the isovaleryl spiramycin I, powdered sugar and dextrin were weighed according to the prescription amount and uniformly mixed. And the above uniformly mixed materials were made into a soft material with a 5% PV-P-$K_{30}$ mucilage, and then the soft material was granulated with a swinging granulation machine, dried at 70°C and subjected to granule dispersion, and the resulting granules were subpackaged after being qualified for inspection.

Embodiment 6 Isovaleryl spiramycin I freeze-dried powder injection

500 mg of isovaleryl spiramycin I raw powder was uniformly mixed with an equimolar amount of adipic acid, and the mixture was dissolved in 5 ml of water to obtain a faint yellow clear solution having a pH between 4.6 and 5.6. Further, 40 mg of mannitol was added as a lyophilized proppant into the faint yellow clear solution, and after being frozen rapidly at a low temperature for 9 hours, the material was freeze-dried to obtain a faint yellow loose mass, which was dissolved in 10 ml of sterile water before being used.

Embodiment 7 Isovaleryl spiramycin II tablets

Specification: 200mg/350mg

Tablet Core Prescription:

| | |
|---|---|
| Isovaleryl spiramycin II | 200g |
| Microcrystalline cellulose | 110g |
| Sodium carboxymethyl starch | 22g |
| Povidone $K_{30}$ (5%) | 15g |

| | |
|---|---|
| Magnesium stearate | 3g |
| Prepared into | 1,000 tablets |
| Coating Liquid Prescription: | |
| Opadry II | 21g |
| Distilled water | appropriate amount |
| Prepared into | 105ml |

Preparation Process:

Preparation of the tablet core: the main drug and adjuvants respectively were made to pass through a 100-mesh sieve, and a prescription amount of isovaleryl spiramycin II, a prescription amount of microcrystalline cellulose and a 1/2 prescription amount of sodium carboxymethyl starch were uniformly mixed, and then a 5% povidone $K_{30}$ aqueous solution was added to make a soft material. A 18-mesh sieve was used for granulating, and the wet granules were dried under a ventilated condition at 60°C for 2 hours. After the wet granules were dried, a 18-mesh sieve was used for dispersing the granules, and then a 1/2 prescription amount of sodium carboxymethyl starch and prescription amount of magnesium

stearate were added. And after the materials were uniformly mixed, the mixture was tabletted with a shallow concave stamping die having a diameter of 11 mm to obtain a tablet core containing drugs, wherein the tablet is 350 mg in weight and 6.5 kg in hardness.

Preparation of the coating liquid: the required Opadry II (white) was weighed, the required amount of water was added into a liquid preparation container, the Opadry II was added into the liquid preparation container in batch. After all the Opadry II was added, the stirring speed was reduced to make the spiral disappear, and then stirring was continued to be performed for 30min to obtain the coating liquid.

Preparation of thin film coated tablets: the tablet core was placed in a coating pan, the coating conditions were determined, and coating was carried out with the host speed of 20 r/min, the inlet air temperature of 40°C, the outlet air temperature of 30°C, the spray pressure of 0.02 Mpa, and the spray slurry flow rate of 1ml/min. And after a constant state was achieved, the coating was continuously to be sprayed for 1.5 hours until the surfaces of the tablets were smooth and uniform in color, wherein tablets which were in compliance with the inspection standard of film coatings were qualified. The coating gains about 5% in weight.

Embodiment 8 (not part of the invention)

Isovaleryl spiramycin II tablets (calculated by 10,000 tablets)

Prescription:

| | |
|---|---|
| Isovaleryl spiramycin II raw powder | 1000g |
| Low-substituted hydroxypropyl | cellulose (5%) 92.5g |
| Sodium carboxymethyl starch | (3%) 55.5g |
| Magnesium stearate (1%) | 18.5g |
| Starch | Total weight minus the weight of other raw and auxiliary materials |
| Total weight | 1,850g |

Preparation process: an appropriate amount of starch was weighed, diluted to a concentration of 15%, and heated to a paste to make a binder. The main material isovaleryl spiramycin II, and the adjuvants starch, low-substituted hydroxypropyl cellulose, sodium carboxymethyl starch, and magnesium stearate were made to pass through a 100-mesh sieve, respectively, and the required main material and adjuvants were weighed according to the prescription amount. After the isovaleryl spiramycin II, starch and low-substituted hydroxypropyl cellulose were fully and uniformly mixed, the starch paste with a starch concentration of 15% was used to prepare the mixture into a soft material which was granulated by a 14-mesh sieve, and granules were dried at 50-60°C to control a water content to be 3-5%. A 14-mesh sieve was used for dispersing the granules, and then sodium carboxymethyl starch and magnesium stearate were added to be mixed, and the granule content was measured. The weight of the tablet was calculated according to the granule content, and the mixture was tabletted (with a Φ9 mm shallow concave punch), then the difference in the weight of the tablets was detected. After passing the test, the tablets were packaged.

Embodiment 9 (not part of the invention)

Isovaleryl spiramycin II capsules (calculated by 10,000 granules)

Prescription:

| | |
|---|---|
| Isovaleryl spiramycin II raw powder | 1,000g |
| Starch | 1,080g minus the weight of isovaleryl spiramycin II raw powder |
| No.3 medicinal capsule | 1,000 granules |
| Liquid paraffin | 50ml |

Preparation process: the main material isovaleryl spiramycin II and the adjuvant medicinal starch were separately weighed according to the process formula amount, and then fully mixed in a mixer for 1.5-2 hours. The data obtained by sampling and content testing should be basically consistent with the theoretical data (the weight of each capsule was about 0.105g), and the qualified No. 3 medicinal capsule and the mixed raw materials to be loaded were filled in a filling device according to the operation requirements of an automatic capsule machine, and the filled capsules were subjected to a difference test (±10% or less, <0.3g) to see if the dissolution rate meets the requirements or not,

the capsules that meet the requirements after being tested were put into a polishing machine to be polished for 15-20 minutes with the liquid paraffin added, and then were taken out to be tested by finished product packaging boxes.

Embodiment 10 (not part of the invention)

Isovaleryl spiramycin II dry syrup (calculated according to 10,000 bags)

Prescription:

| | |
|---|---|
| Isovaleryl spiramycin II raw powder | 1,250g |
| Citric acid (0.5%) | 15g |
| Sucrose | total weight minus other raw and auxiliary materials |
| Total weight | about 500g |
| Pigment (curcumin) | about 1g |

Preparation process: the isovaleryl spiramycin II raw powder, citric acid and sucrose were respectively grinded into granules by a high-speed jet mill, and 85% of the granules pass through a 300-mesh sieve, 15% of the granules pass through a 180-mesh sieve. Then the fine powder after grinding was weighed according to the prescription amount and fully mixed for 1-1.5 hours, the content was measured, the loading capacity was calculated (the theoretical loading capacity was 500mg per bag). Then the mixture was put into a bagging machine, aluminum foil paper was installed, and filling was carried out according to the operation requirements of a filling machine. The difference was allowed to be within ±5 %, and after the filling, the outer packaging was carried out after passing the inspection.

Embodiment 11 (not part of the invention)

Isovaleryl spiramycin II granules (calculated according to 10,000 bags)

Prescription:

| | |
|---|---|
| Isovaleryl spiramycin II raw powder | 1,250g |
| Powdered sugar | 20,000g |
| Dextrin | 9,000g |

Preparation process: the isovaleryl spiramycin II raw powder, powdered sugar and dextrin were made to pass through a 120-mesh sieve, and the isovaleryl spiramycin II, powdered sugar and dextrin were weighed according to the prescription amount and uniformly mixed. The above uniformly mixed materials were made into a soft material with a 5% PV-P-$K_{30}$ mucilage, and then the soft material was granulated with a swinging granulation machine, dried at 70°C and subjected to granule dispersion, and the resulting granules were subpackaged after being qualified for inspection.

Embodiment 12 (not part of the invention)

Isovaleryl spiramycin II freeze-dried powder injection

500 mg of isovaleryl spiramycin II raw powder was weighed and uniformly mixed with an equimolar amount of adipic acid, and the mixture was dissolved in 5 ml of water to obtain a faint yellow clear solution having a pH between 4.6 and 5.6. Further, 40 mg of mannitol was added as a lyophilized proppant into the faint yellow clear solution, and after being frozen rapidly at a low temperature for 9 hours, the material was freeze-dried to obtain a faint yellow loose mass, which was dissolved in 10 ml of sterile water before being used.

Embodiment 13 Isovaleryl spiramycin III tablets

Specification: 200mg/350mg

Tablet Core Prescription:

| | |
|---|---|
| Isovaleryl spiramycin III | 200g |
| Microcrystalline cellulose | 110g |

(continued)

| | |
|---|---|
| Sodium carboxymethyl starch | 22g |
| Povidone $K_{30}$ (5%) | 15g |
| Magnesium stearate | 3g |
| Prepared into | 1,000 tablets |

Coating Liquid Prescription:

| | |
|---|---|
| Opadry II | 21g |
| Distilled water | appropriate amount |
| Prepared into | 105ml |

Preparation Process:

Preparation of the tablet core: the main drug and adjuvants respectively were made to pass through a 100-mesh sieve, and a prescription amount of isovaleryl spiramycin III, a prescription amount of microcrystalline cellulose and a 1/2 prescription amount of sodium carboxymethyl starch were uniformly mixed, and then a 5% povidone $K_{30}$ aqueous solution was added to make a soft material. A 18-mesh sieve was used for granulating, and the wet granules were dried under a ventilated condition at 60°C for 2 hours. After the wet granules were dried, a 18-mesh sieve was used for dispersing the granules, and then a 1/2 prescription amount of sodium carboxymethyl starch and prescription amount of magnesium stearate were added. And after the materials were uniformly mixed, the mixture was tabletted compressed with a shallow concave stamping die having a diameter of 11 mm to obtain a tablet core containing drugs, wherein the tablet is 350 mg in weight and 6.5 kg in hardness.

Preparation of the coating liquid: the required Opadry II (white) was weighed, the required amount of water was added into a liquid preparation container, the Opadry II was added into the liquid preparation container in batch. After all the Opadry II was added, the stirring speed was reduced to make the spiral disappear, and then stirring was continued to be performed for 30min to obtain the coating liquid.

Preparation of thin film coated tablets: the tablet core was placed in a coating pan, the coating conditions were determined, and coating was carried out with the host speed of 20 r/min, the inlet air temperature of 40°C, the outlet air temperature of 30°C, the spray pressure of 0.02 Mpa, and the spray slurry flow rate of 1ml/min. And after a constant state was achieved, the coating was continuously to be sprayed for 1.5 hours until the surfaces of the tablets were smooth and uniform in color, wherein tablets which were in compliance with the inspection standard of film coatings were qualified. The coating gains about 5% in weight.

Embodiment 14 Isovaleryl spiramycin III tablets (calculated by 10,000 tablets)

Prescription:

| | |
|---|---|
| Isovaleryl spiramycin III raw powder | 1000g |
| Low-substituted hydroxypropyl cellulose (5%) | 92.5g |
| Sodium carboxymethyl starch | (3%) 55.5g |
| Magnesium stearate (1%) | 18.5g |
| Starch | Total weight minus the weight of other raw and auxiliary materials |

| | |
|---|---|
| Total weight | 1,850g |

Preparation process: an appropriate amount of starch was weighed, diluted to a concentration of 15%, and heated to a paste to get a binder. The main material isovaleryl spiramycin III, and the adjuvants starch, low-substituted hydroxypropyl cellulose, sodium carboxymethyl starch, and magnesium stearate were made to pass through a 100-mesh sieve, respectively, and the required main material and adjuvants were weighed according to the prescription amount. After the isovaleryl spiramycin III, starch and low-substituted hydroxypropyl cellulose were fully and uniformly mixed, the starch paste with a starch concentration of 15% was used to prepare the mixture into a soft material which was granulated by a 14-mesh sieve, and granules were dried at 50-60°C to control a water content to be 3-5%. A 14-mesh sieve was used for dispersing the granules, and then sodium carboxymethyl starch and magnesium stearate were

added to be mixed, and the granule content was measured;. The weight of the tablet was calculated according to the granule content, and the mixture was tabletted (with a Φ9 mm shallow concave punch), then the difference in the weight of the tablets was detected. After passing the test, the tablets were packaged.

Embodiment 15 Isovaleryl spiramycin III capsules (calculated by 10,000 granules)

Prescription:

| | |
|---|---|
| Isovaleryl spiramycin III raw powder | 1,000g |
| Starch | 1,080g minus the weight of isovaleryl spiramycin III raw powder |
| No.3 medicinal capsule | 1,000 granules |
| Liquid paraffin | 50ml |

Preparation process: the main material isovaleryl spiramycin III and the adjuvant medicinal starch were separately weighed according to the process formula amount, and then fully mixed in a mixer for 1.5-2 hours. The data obtained by sampling and content testing should be basically consistent with the theoretical data (the weight of each capsule was about 0.105g), and the qualified No. 3 medicinal capsule and the mixed raw materials to be loaded were filled in a filling device according to the operation requirements of an automatic capsule machine, and the filled capsules were subjected to a difference test (±10% or less, <0.3g) to see if the dissolution rate meets the requirements or not, the capsules that meet the requirements after being tested were put into a polishing machine to be polished for 15-20 minutes with the liquid paraffin added, and then were taken out to be tested by finished product packaging boxes.

Embodiment 16 Isovaleryl spiramycin III dry syrup (calculated according to 10,000 bags)

Prescription:

| | |
|---|---|
| Isovaleryl spiramycin III raw powder | 1,250g |
| Citric acid (0.5%) | 15g |
| Sucrose | total weight minus other raw and auxiliary materials |
| Total weight | about 500g |
| Pigment (curcumin) | about 1g |

Preparation process: the isovaleryl spiramycin III raw powder, citric acid and sucrose were respectively grinded into granules by a high-speed jet mill, and 85% of the granules pass through a 300-mesh sieve, 15% of the granules pass through a 180-mesh sieve. And then the fine powder after grinding was weighed according to the prescription amount and fully mixed for 1-1.5 hours, the content was measured, the loading capacity was calculated (the theoretical loading capacity was 500mg per bag). Then the mixture was put into a bagging machine, aluminum foil paper was installed, and filling was carried out according to the operation requirements of a filling machine. The difference was allowed to be within ±5 %, and after the filling, the outer packaging was carried out after passing the inspection.

Embodiment 17 Isovaleryl spiramycin III granules (calculated according to 10,000 bags)

Prescription:

| | |
|---|---|
| Isovaleryl spiramycin III raw powder | 1,250g |
| Powdered sugar | 20,000g |
| Dextrin | 9,000g |
| 5% PV-P-$K_{30}$ | appropriate amount |

Preparation process: the isovaleryl spiramycin III raw powder, powdered sugar and dextrin were made to pass through a 120-mesh sieve, and the isovaleryl spiramycin III, powdered sugar and dextrin were weighed according to the prescription amount and uniformly mixed. The above uniformly mixed materials were made into a soft material with a 5% PV-P-$K_{30}$ mucilage, and then the soft material was granulated with a swinging granulation machine, dried at 70°C and subjected to granule dispersion, and the resulting granules were subpackaged after being qualified for inspection.

Embodiment 18 Isovaleryl spiramycin III freeze-dried powder injection

500 mg of isovaleryl spiramycin III raw powder was uniformly mixed with an equimolar amount of adipic acid, and the mixture was dissolved in 5 ml of water to obtain a faint yellow clear solution having a pH between 4.6 and 5.6. Further, 40 mg of mannitol was added as a lyophilized proppant into the faint yellow clear solution, and after being frozen rapidly at a low temperature for 9 hours, the material was freeze-dried to obtain a faint yellow loose mass, which was dissolved in 10 ml of sterile water before being used.

**Test Example 1 Bioassay of anti-tumor activity**

The purpose of the assay is to evaluate the in vitro cell proliferation inhibition or cytotoxic activity of a tested sample.

Cell strains:

Human breast cancer cells MCF-7 and MDA-MB-231, human hepatoma cells HepG2, human non-small cell lung cancer cells A549, human large cell lung cancer cells H460 and H1299, human renal clear cell adenocarcinoma cell 786-O, human renal cell adenocarcinoma cell 769-P, human glioma cell U251, human glioblastoma cell A172, human tissue lymphoma cell U937, human cervical cancer cell HeLa, human prostate cancer cell PC3, human pancreatic cancer cell PANC-1, human esophageal cancer cell TE-1, human gastric adenocarcinoma cell SGC7901, human colon cancer cell HT-29, human promyelocytic leukemia cell HL-60, human thyroid cancer cell TPC-1, and human bladder cancer cell T-24.

Reagents:

RPMI1640 medium, MEM medium, DMEM low sugar medium, fetal calf serum purchased from Gibco, USA, trypsin, glutamine, penicillin, streptomycin, dimethyl sulfoxide (DMSO), and methyl-thiazol-tetrazolium (MTT) purchased from Sigma, USA.

Instruments:

Carbon dioxide incubator (Sanyo, Japan), enzyme-linked immunosorbent analyzer (Tecan, Austria), 96-well culture plate (Corning, USA), inverted microscope (Motic, China).

The operation steps are as follows:

Adherent cells:

MCF-7, MDA-MB-231, HepG2, A549, H460, H1299, 786-O, 769-P, U251, A172, HeLa, PC3, PANC-1, TE-1, SGC7901, and HT-29 were adherent tumor cells. The adherent tumor cells in the logarithmic growth phase were selected and digested with trypsin, then were prepared into a 4 to $5\times10^4$/ml cell suspension by a medium containing 10% fetal bovine serum. And the cell suspension was inoculated in a 96-well culture plate, and each well was 100 μL. The 96-well culture plate was cultured at 37°C and 5% $CO_2$ for 24 hours. The experimental group was replaced with a new culture medium containing different concentrations of the sample to be tested, namely, isovaleryl spiramycin I, isovaleryl spiramycin II, or isovaleryl spiramycin III, while the control group was replaced with a culture medium containing the same volume of solvent. Each group was set up with 3 parallel wells that were cultured at 37°C and 5% $CO_2$ for 48 hours. After the supernatant was removed, the wells were washed carefully for 3 times with PBS. And 100 μL of freshly prepared culture medium containing 0.5 mg/ml MTT was added to each well for continuous incubation for 4 hours at 37°C. After the supernatant was removed carefully, 150 μL of DMSO was added to each well, and after the material was mixed for 10 minutes with a micro-oscillator, the optical density value was measured at 492 nm with a microplate reader.

Suspension cells:

U937 and HL-60 were suspension cells, and cells in a logarithmic growth phase were selected and prepared into a $2\times10^5$/ml cell suspension by a RPMI 1640 culture medium containing 10% fetal bovine serum. And the cell suspension was inoculated in a 96-well culture plate, and each well was 50 μL and the 96-well culture plate was cultured at 37°C and 5% $CO_2$ for 24 hours. 50 μL of a culture medium containing different concentrations of the tested sample isovaleryl spiramycin I, isovaleryl spiramycin II, or isovaleryl spiramycin III was added in the experimental group, while a culture medium containing the same volume of solvent was added into the control group. Each group was set up with 3 parallel wells that were cultured at 37°C and 5% $CO_2$ for 48 h. And 10 μL of freshly prepared medium containing 5 mg/ml MTT was added into each well for continuous incubation for 4 hours at 37°C. The crystals were dissolved in 100 μL of a triple solution (SDS 10 g, 10 M HCl 0.1 mL, isobutanol 5 mL, diluted with distilled water to 100 mL), and incubated at 37°C for 12 hours. The optical density value was measured at 492 nm with a microplate reader.

Evaluation of results:

The inhibition rate of the medicament on tumor cell growth is calculated according to the following formula:

$$\text{Tumor cell growth inhibition rate (\%)} = [A_{492}\text{(negative control)} - A_{492}\text{(dosing group)}]/A_{492}\text{(negative control)} \times 100\%$$

And the half-inhibitory concentration ($IC_{50}$) of the sample is determined therefrom.

Results:

The evaluation results of in vitro antiproliferative activity of the samples selected from human breast cancer cells MCF-7 and MDA-MB-231, human hepatoma cells HepG2, human non-small cell lung cancer cells A549, human large cell lung cancer cells H460 and H1299, human renal clear cell adenocarcinoma cell 786-O, human renal cell adenocarcinoma cell 769-P, human glioma cell U251, human glioblastoma cell A172, human tissue lymphoma cell U937, human cervical cancer cell HeLa, human prostate cancer cell PC3, human pancreatic cancer cell PANC-1, human esophageal cancer cell TE-1, human gastric adenocarcinoma cell SGC7901, human colon cancer cell HT-29, human promyelocytic leukemia cell HL-60, human thyroid cancer cell TPC-1, and human bladder cancer cell T-24 are shown in Table 1, Table 2 and Table 3:

Table 1. Inhibition of isovaleryl spiramycin I on the proliferation of tumor cells

| Cell Strain | $IC_{50}$ (μg/mL) | Cell Strain | $IC_{50}$ (μg/mL) |
|---|---|---|---|
| MCF-7 | 20.79±1.57 | A172 | 10.24±0.37 |
| MDA-MB-231 | 18.12±0.61 | U937 | 10.88±0.05 |
| HepG2 | 17.90±1.74 | HeLa | 10.31±0.27 |
| A549 | 19.93±1.66 | PC3 | 9.39±0.85 |
| H460 | 19.31±0.35 | PANC-1 | 9.96±0.46 |
| H1299 | 24.03±2.07 | TE-1 | 8.42±1.53 |
| 786-O | 5.08±0.08 | SGC-7901 | 11.28±1.27 |
| 769-P | 5.09±0.04 | HT-29 | 17.30±0.52 |
| U251 | 11.01±0.32 | HL-60 | 16.52±1.26 |
| TPC-1 | 19.97±1.93 | T-24 | 18.68±0.58 |

Table 2. Inhibition of isovaleryl spiramycin II on the proliferation of tumor cells (for reference only)

| Cell Strain | $IC_{50}$ (μg/mL) | Cell Strain | $IC_{50}$ (μg/mL) |
|---|---|---|---|
| MCF-7 | 51.14±2.54 | A172 | 33.62±0.57 |
| MDA-MB-231 | 49.60±0.39 | U937 | 34.92±0.81 |
| HepG2 | 37.94±1.71 | HeLa | 33.31±0.40 |
| A549 | 36.97±2.92 | PC3 | 32.88±0.53 |
| H460 | 41.16±0.71 | PANC-1 | 31.93±0.12 |
| H1299 | 42.24±0.44 | TE-1 | 35.59±2.64 |
| 786-O | 20.18±0.86 | SGC-7901 | 39.14±1.21 |
| 769-P | 20.61±0.48 | HT-29 | 31.05±3.23 |
| U251 | 35.35±1.57 | HL-60 | 29.39±1.82 |
| TPC-1 | 45.37±2.90 | T-24 | 39.85±3.26 |

Table 3. Inhibition of isovaleryl spiramycin III on the proliferation of tumor cells

| Cell Strain | IC50(μg/mL) | Cell Strain | IC50(μg/mL) |
|---|---|---|---|
| MCF-7 | 32.44±1.18 | A172 | 16.17±0.54 |
| MDA-MB-231 | 29.47±0.28 | U937 | 15.59±0.08 |
| HepG2 | 27.42±3.52 | HeLa | 15.86±0.62 |
| A549 | 28.84±1.73 | PC3 | 16.37±0.27 |
| H460 | 28.80±0.19 | PANC-1 | 13.18±0.23 |
| H1299 | 32.40±0.41 | TE-1 | 20.19±1.83 |
| 786-O | 10.52±0.56 | SGC-7901 | 17.26±0.79 |
| 769-P | 10.75±0.56 | HT-29 | 22.68±2.32 |
| U251 | 16.46±1.54 | HL-60 | 21.38±1.75 |
| TPC-1 | 29.72±4.02 | T-24 | 31.23±1.47 |

The available results show that the samples isovaleryl spiramycin I, isovaleryl spiramycin II, and isovaleryl spiramycin III show good anti-proliferative activity against the cells tested.

Test Example 2 In Vivo Test

**1. Inhibition of isovaleryl spiramycin I, II and III on human large cell lung cancer cells H460 in nude mice model**

**Establishment of a mouse solid tumor model**

H460 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = V/Vo (Vo is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 4, and Table 5).

The tumor growth inhibition rates of isovaleryl spiramycin I groups with low, medium and high doses are 20.70%,

46.33% and 70.11%, respectively.

The tumor growth inhibition rates of isovaleryl spiramycin II groups with low, medium and high doses are 14.22%, 34.43% and 61.12%, respectively.

The tumor growth inhibition rates of isovaleryl spiramycin III groups with low, medium and high doses are 17.41%, 23.31% and 63.93%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 56.56%, 49.00% and 31.96% respectively.

The relative tumor proliferation rates of isovaleryl spiramycin II groups with low, medium and high doses are 51.97%, 46.49% and 37.89% respectively.

The relative tumor proliferation rates of isovaleryl spiramycin III groups with low, medium and high doses are 63.03%, 42.54% and 35.95% respectively.

There are no significant changes in the mouse weight of isovaleryl spiramycin I, II and III groups with low, medium and high doses compared with the model group.

Table 4. Effect of isovaleryl spiramycin I, II (for reference) and III on the inhibition rate of transplanted tumor of human large cell lung cancer H460 cells in nude mice ($x \pm s$)

| Group | Dose (mg/kg) | Number of Animals (Start/End) | Body Weight (g)(dl) | Body Weight (g)(d30) | Tumor Weight (g) | Inhibition Rate(%) |
|---|---|---|---|---|---|---|
| Model Group | 0 | 6/6 | 20.47±0.41 | 24.53±1.74 | 1.72±0.18 | -- |
| Cyclophosphamide | 30 | 6/6 | 20.46±0.54 | 21.20±0.75 | 0.50±0.06*** | 71.08 |
| Isovaleryl spiramycin I | 25 | 6/6 | 20.67±0.33 | 22.55±1.90 | 1.37±0.13** | 20.70 |
| | 50 | 6/6 | 20.45±0.62 | 23.54±1.18 | 0.93±0.18*** | 46.33 |
| | 100 | 6/6 | 20.58±0.32 | 23.91±1.34 | 0.52±0.07*** | 70.11 |
| Isovaleryl | 25 | 6/6 | 20.72±0.47 | 24.10±1.02 | 1.48±0.24 | 14.22 |
| spiramycin II | 50 | 6/6 | 20.58±0.83 | 22.49±2.46 | 1.13±0.08** | 34.43 |
| | 100 | 6/6 | 20.61±0.69 | 24.87±0.74 | 0.67±0.20*** | 61.12 |
| Isovaleryl spiramycin III | 25 | 6/6 | 20.39±0.56 | 25.09±1.38 | 1.42±0.26* | 17.41 |
| | 50 | 6/6 | 20.56±0.47 | 24.94±0.68 | 1.32±0.25* | 23.31 |
| | 100 | 6/6 | 20.56±0.64 | 25.07±1.48 | 0.62±0.26*** | 63.93 |
| *p<0.05 compared with the model group, **p<0.01 compared with the model group, ***p<0.001 compared with the model group | | | | | | |

Table 5. Effect of isovaleryl spiramycin I, II and III on the volume change of transplanted tumor of human large cell lung cancer H460 cells in nude mice (x±s)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferation Rate (T/C) |
|---|---|---|---|---|---|---|
| Model Group | 0 | 6/6 | 146.29±28.15 | 1497.79±178.37 | 10.51±1.89 | |
| Cyclophosphamide | 30 | 6/6 | 146.98±27.55 | 468.49±128.57*** | 3.24±0.99*** | 30.86 |
| Isovaleryl spiramycin I | 25 | 6/6 | 148.20±15.93 | 889.44±343.94** | 5.94±2.02** | 56.56 |
| | 50 | 6/6 | 148.51±17.68 | 753.94±306.82** | 5.15±2.25** | 49.00 |
| | 100 | 6/6 | 145.08±19.59 | 477.54±89.21*** | 3.36±0.85*** | 31.96 |
| Isovaleryl spiramycin II | 25 | 6/6 | 147.44±15.40 | 804.17±292.57 | 5.46±1.87 | 51.97 |
| | 50 | 6/6 | 146.77±18.26 | 700.40±143.83** | 4.88±1.36** | 46.49 |
| | 100 | 6/6 | 149.76±13.12 | 604.38±195.98*** | 3.98±1.10*** | 37.89 |
| Isovaleryl spiramycin III | 25 | 6/6 | 140.44±14.04 | 914.05±279.14** | 6.62±2.34* | 63.03 |
| | 50 | 6/6 | 148.59±14.47 | 666.74±160.61*** | 4.47±0.94*** | 42.54 |
| | 100 | 6/6 | 143.19±17.40 | 531.56±78.33*** | 3.78±0.84*** | 35.95 |
| *p<0.05 compared with the model group; **p<0.01 compared with the model group, ***p<0.001 compared with the model group | | | | | | |

**2. Inhibition of isovaleryl spiramycin I, II and III on human non-small cell lung cancer cell H1299 in nude mice model**

**Establishment of a mouse solid tumor model**

H1299 cells in a logarithmic growth phase were taken and subjected to a trypan blue exclusion experiment showing that the cell viability was more than95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = V/Vo (Vo is the tumor volume before administration, V is the tumor volume before sacrifice), and T/C (%) = treatment group RTV/model control group RTV$\times$100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) $\times$ 100%.

The test results show that compared with the control group, each drug-administered group has a certain degree of inhibition on the tumor growth inhibition rate, tumor volume, relative tumor volume and relative tumor proliferation rate (see Table 6, and Table 7).

The tumor growth inhibition rates of isovaleryl spiramycin I groups with low, medium and high doses are 22.11%, 43.83% and 69.95%, respectively.

The tumor growth inhibition rates of isovaleryl spiramycin II groups with low, medium and high doses are 17.32%, 44.21% and 58.37%, respectively.

The tumor growth inhibition rates of isovaleryl spiramycin III groups with low, medium and high doses are 13.11%, 49.38% and 62.78%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 89.42%, 49.81% and 27.43% respectively.

The relative tumor proliferation rates of isovaleryl spiramycin II groups with low, medium and high doses are 83.01%, 46.94% and 36.86% respectively.

The relative tumor proliferation rates of isovaleryl spiramycin III groups with low, medium and high doses are 89.88%, 48.11% and 32.43% respectively.

There are no significant changes in the mouse weight of isovaleryl spiramycin I, II and III groups with low, medium and high doses compared with the model group.

Table 6. Effect of isovaleryl spiramycin I, II and III on the inhibition rate of transplanted tumor of human non-small cell lung cancer cell H1299 in nude mice (x±s)

| Group | Dose (mg/kg) | Number of Animals (Start/End) | BodyWeight(g) (dl) | Body Weight (g) (d30) | Tumor Weight (g) | Inhibition Rate (%) |
|---|---|---|---|---|---|---|
| Model Group | 0 | 6/6 | 21.85±0.77 | 27.73±1.52 | 1.74±0.21 | |

(continued)

| Group | Dose (mg/kg) | Number of Animals (Start/End) | BodyWeight(g) (dl) | Body Weight (g) (d30) | Tumor Weight (g) | Inhibition Rate (%) |
|---|---|---|---|---|---|---|
| Cyclophos phamide | 30 | 6/6 | 21.83±0.33 | 23.72±2.41 | 0.38±0.08*** | 78.00 |
| Isovaleryl spiramycin I | 25 | 6/6 | 21.82±0.41 | 28.49±4.23 | 1.36±0.33* | 22.11 |
| | 50 | 6/6 | 21.74±1.04 | 27.99±2.47 | 0.98±0.27*** | 43.83 |
| | 100 | 6/6 | 21.64±0.96 | 27.62±2.63 | 0.52±0.21*** | 69.95 |
| Isovaleryl spiramycin II | 25 | 6/6 | 21.91±0.85 | 28.06±2.64 | 1.44±0.29* | 17.32 |
| | 50 | 6/6 | 21.99±1.18 | 25.65±5.03 | 0.97±0.16*** | 44.21 |
| | 100 | 6/6 | 21.54±0.88 | 28.81±1.21 | 0.73±0.25*** | 58.37 |
| Isovaleryl spiramycin III | 25 | 6/6 | 21.73±0.32 | 28.91±1.32 | 1.51±0.30 | 13.11 |
| | 50 | 6/6 | 21.82±0.53 | 27.84±2.73 | 0.88±0.20*** | 49.38 |
| | 100 | 6/6 | 21.77±0.58 | 27.71±1.48 | 0.65±0.17*** | 62.78 |
| *p<0.05 compared with the model group, **p<0.01 compared with the model group, ***p<0.001 compared with the model group | | | | | | |

Table 7. Effect of isovaleryl spiramycin I, II and III on the volume change of transplanted tumor of human non-small cell lung cancer cell H1299 in nude mice (x±s)

| Group | Dose (mg/kg) | Number of Animals (Start/En d) | Tumor Volume (mm3) (d1) | Tumor Volume (mm3) (d30) | Relative Tumor Volume (RTV) | Relative Tumor Prolifera tion Rate (T/C) |
|---|---|---|---|---|---|---|
| Model Group | 0 | 6/6 | 136.68±18.16 | 1710±163.11 | 12.82±2.82 | |
| Cyclophos phamide | 30 | 6/6 | 135.46±16.48 | 384.21±106.99 | 2.91±0.95*** | 22.72 |
| Isovaleryl spiramycin I | 25 | 6/6 | 136.07±17.13 | 1513.14±387.19* | 11.46±3.83 | 89.42 |
| | 50 | 6/6 | 135.51±20.47 | 861.71±164.32*** | 6.39±1.01** | 49.81 |
| | 100 | 6/6 | 137.34±22.95 | 470.36±21.77*** | 3.52±0.68*** | 27.43 |
| Isovaleryl spiramycin II | 25 | 6/6 | 139.13±12.26 | 1474.57±104.54 | 10.64±0.92 | 83.01 |
| | 50 | 6/6 | 138.94±20.16 | 821.57±90.36*** | 6.02±1.07*** | 46.94 |
| | 100 | 6/6 | 136.94±14.60 | 634.05±180.18*** | 4.72±1.57*** | 36.86 |
| Isovaleryl spiramycin III | 25 | 6/6 | 137.58±14.95 | 1565.97±277.97 | 11.52±2.51 | 89.88 |
| | 50 | 6/6 | 137.82±17.12 | 816.87±299.03** | 6.17±2.64*** | 48.11 |
| | 100 | 6/6 | 137.09±12.95 | 557.82±209.73*** | 4.16±1.90*** | 32.43 |
| *p<0.05 compared with the model group; **p<0.01 compared with the model group, ***p<0.001 compared with the model group | | | | | | |

### 3. Inhibition of isovaleryl spiramycin I, II and III on human esophageal cancer in nude mice model

#### Establishment of a mouse solid tumor model

TE-1 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

#### Calculation of tumor volume and relative tumor proliferation rate

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = V/Vo (Vo is the tumor volume before administration, V is the tumor volume before sacrifice), and T/C (%) = treatment group RTV/model control group RTV$\times$100%.

#### Calculation of tumor growth inhibition rate

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) $\times$ 100%.

The test results show that compared with the control group, each drug-administered group has a certain degree of inhibition on the tumor growth inhibition rate, tumor volume, relative tumor volume and relative tumor proliferation rate (see Table 8, and Table 9).

The tumor growth inhibition rates of isovaleryl spiramycin I groups with low, medium and high doses are 30.92%, 51.01% and 69.71%, respectively.

The tumor growth inhibition rates of isovaleryl spiramycin II groups with low, medium and high doses are 24.87%, 43.78% and 72.48%, respectively.

The tumor growth inhibition rates of isovaleryl spiramycin III groups with low, medium and high doses are 22.64%, 40.17% and 65.46%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 60.55%, 40.70% and 20.61% respectively.

The relative tumor proliferation rates of isovaleryl spiramycin II groups with low, medium and high doses are 70.32%, 50.51% and 36.49% respectively.

The relative tumor proliferation rates of isovaleryl spiramycin III groups with low, medium and high doses are 66.52%, 50.71% and 30.72% respectively.

There are no significant changes in the mouse weight of isovaleryl spiramycin I, II and III groups with low, medium and high doses compared with the model group.

Table 8. Effect of isovaleryl spiramycin I, II and III on the inhibition rate of transplanted tumor of human esophageal cancer cell TE-1 in nude mice (x±s)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 22.67 ±0.87 | 20.97 ±0.97 | 1.57 ± 0.62 | - |
| Cyclophos phamide | 30 | 6/6 | 23.18 ±1.22 | 20.57 ±1.14 | 0.44 ± 0.18 ** | 71.87 |
| Isovaleryl spiramycin I Isovaleryl spiramycin II | 12.5 | 6/6 | 23.27 ±1.35 | 21.75 ±0.60 | 1.08 ± 0.24 | 30.92 |
| | 25 | 6/6 | 22.98 ±1.83 | 21.28 ±1.04 | 0.77 ± 0.43 * | 51.01 |
| | 50 | 6/6 | 22.52 ±1.19 | 22.32 ±0.52 | 0.48 ± 0.36 ** | 69.71 |
| | 12.5 | 6/6 | 21.43 ±1.58 | 21.97 ±0.99 | 1.18 ± 0.60 | 24.87 |
| | 25 | 6/6 | 22.93 ±0.41 | 22.28 ±0.66 | 0.88 ± 0.46 | 43.78 |
| | 50 | 6/6 | 22.35 ±1.07 | 21.62 ±0.32 | 0.43 ± 0.26 ** | 72.48 |
| Isovaleryl spiramycin III | 12.5 | 6/6 | 22.52 ±0.57 | 21.38 ±0.37 | 1.21 ± 0.53 | 22.64 |
| | 25 | 6/6 | 21.82 ±1.34 | 21.38 ±0.67 | 0.94 ± 0.39 | 40.17 |
| | 50 | 6/6 | 21.23 ±1.05 | 20.70 ±0.63 | 0.54 ± 0.22 ** | 65.46 |
| *p<0.05 compared with the model group, **p<0.01 compared with the model group, ***p<0.001 compared with the model group | | | | | | |

Table 9. Effect of isovaleryl spiramycin I, II and III on the volume change of transplanted tumor of human esophageal cancer cell TE-1 in nude mice (x±s)

| Group | Dose (mg/kg) | Number of Animals (Start/En d) | Tumor Volume (mm3) (d1) | Tumor Volume (mm3) (d30) | Relative Tumor Volume (RTV) | Relative Tumor Prolifera tion Rate (T/C) |
|---|---|---|---|---|---|---|
| Model Group | 0 | 6/6 | 218.70 ±76.83 | 2378.49±829.69 *** | 11.89±15.80*** | -- |
| Cyclophosp hamide | 30 | 6/6 | 269.96±92.18 | 886.22±271.22 *** | 4.00±1.91 *** | 30.18 |
| Isovaleryl spiramycin I | 12.5 | 6/6 | 408.51 ±150.80 | 2690.27±374.79 *** | 5.48±3.80*** | 60.55 |
| | 25 | 6/6 | 258.44 ±104.69 | 1143.85±402.36** | 3.94±2.71** | 40.70 |
| | 50 | 6/6 | 279.56 ±156.70 | 626.64±431.35 | 3.65±1.71 | 20.61 |

(continued)

| Group | Dose (mg/kg) | Number of Animals (Start/En d) | Tumor Volume (mm3) (d1) | Tumor Volume (mm3) (d30) | Relative Tumor Volume (RTV) | Relative Tumor Prolifera tion Rate (T/C) |
|---|---|---|---|---|---|---|
| Isovaleryl spiramycin II | 12.5 | 6/6 | 134.33±57.77 | 1027.37±363.55 ** | 7.92±6.86** | 70.32 |
| | 25 | 6/6 | 195.76±76.95 | 1075.36±720.20* | 6.99±5.39* | 50.51 |
| | 50 | 6/6 | 143.85±16.25 | 570.92±293.97* | 6.97±7.85* | 36.49 |
| Isovaleryl spiramycin III | 12.5 | 6/6 | 173.45±63.55 | 1254.78± 637.73** | 6.03±5.27** | 66.52 |
| | 25 | 6/6 | 206.72±79.72 | 1140.10 ±819.08* | 5.02±6.81* | 50.71 |
| | 50 | 6/6 | 331.09 ±208.02 | 1106.23±865.11 | 3.60±1.29 | 30.72 |
| *p<0.05 compared with the model group; **p<0.01 compared with the model group, ***p<0.001 compared with the model group | | | | | | |

**4. Inhibition of isovaleryl spiramycin I, II and III on human gastric adenocarcinoma in nude mice model**

**Establishment of a mouse solid tumor model**

SGC7901 cells in a logarithmic growth phase were taken and subjected to a trypan blue exclusion experiment showing that the cell viability is was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = V/Vo (Vo is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 10, and Table 11).

The tumor growth inhibition rates of isovaleryl spiramycin I groups with low, medium and high doses are 31.56%, 53.13% and 70.78%, respectively.

The tumor growth inhibition rates of isovaleryl spiramycin II groups with low, medium and high doses are 24.44%, 41.76% and 70.24%, respectively.

The tumor growth inhibition rates of isovaleryl spiramycin III groups with low, medium and high doses are 21.68%, 41.13% and 63.34%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are 61.13%, 42.67% and 20.23% respectively.

The relative tumor proliferation rates of isovaleryl spiramycin II groups with low, medium and high doses are 70.48%, 51.42% and 36.95% respectively.

The relative tumor proliferation rates of isovaleryl spiramycin III groups with low, medium and high doses are 65.48%, 49.44% and 30.34% respectively.

There are no significant changes in the mouse weight of isovaleryl spiramycin I, II and III groups with low, medium and high doses compared with the model group.

Table 10. Effect of isovaleryl spiramycin I, II and III on the inhibition rate of transplanted tumor of human gastric adenocarcinoma cell SGC7901 in nude mice (x±s)

| Group | Dose (mg/kg) | Number of Animals (Start/En d) | Body Weight (g) (d1) | Body Weight (g) (d30) | Tumor Weight (g) | Inhibition Rate (%) |
|---|---|---|---|---|---|---|
| Model Group | 0 | 6/6 | 22.38 ±0.71 | 21.08±0.76 | 1.97 ± 0.61 | -- |
| Cyclophos phamide | 30 | 6/6 | 22.63 ±1.35 | 19.85±0.72 | 0.44 ± 0.41*** | 72.26 |
| Isovaleryl spiramycin I | 12.5 | 6/6 | 22.02 ±0.76 | 21.63 ±0.80 | 1.07 ± 0.53 * | 31.56 |
| | 25 | 6/6 | 22.05 ±1.55 | 21.12 ±0.90 | 0.74 ± 0.52 ** | 53.13 |
| | 50 | 6/6 | 22.65 ±0.95 | 21.87 ±0.61 | 0.46 ± 0.36 *** | 70.78 |
| Isovaleryl spiramycin II | 12.5 | 6/6 | 22.45 ±1.20 | 21.73 ±0.98 | 1.19 ± 0.62 | 24.44 |
| | 25 | 6/6 | 22.65 ±0.81 | 22.12 ±0.85 | 0.91 ± 0.53 ** | 41.76 |
| | 50 | 6/6 | 22.13 ±1.35 | 21.40 ±0.38 | 0.47 ± 0.42 *** | 70.24 |
| Isovaleryl spiramycin III | 12.5 | 6/6 | 22.68 ±0.45 | 21.55 ±0.63 | 1.23 ± 0.70 | 21.68 |
| | 25 | 6/6 | 21.98 ±1.37 | 21.55 ±0.95 | 0.92 ± 0.40 ** | 41.13 |
| | 50 | 6/6 | 21.42 ±1.10 | 20.57 ±0.87 | 0.58 ± 0.52 ** | 63.34 |
| *p<0.05 compared with the model group, **p<0.01 compared with the model group, ****p<0.001 compared with the model group | | | | | | |

Table 11. Effect of isovaleryl spiramycin I, II and III on the volume change of transplanted tumor of human gastric adenocarcinoma cell SGC7901 in nude mice (x±s)

| Group | Dose (mg/kg) | Number of Animals (Start/End) | Tumor Volume (mm3) (dl) | Tumor Volume (mm3) (d30) | Relative Tumor Volume (RTV) | Relative Tumor Proliferation Rate(T/C) |
|---|---|---|---|---|---|---|
| Model Group | 0 | 6/6 | 212.82 ±68.03 | 2373.38 ±834.85*** | 11.15±12.27*** | -- |
| Cyclophosphamide | 30 | 6/6 | 252.63 ±74.57 | 865.27±422.15* | 4.27±2.36* | 30.71 |
| Isovaleryl spiramycin I | 12.5 | 6/6 | 412.96±157.62 | 2815.50 ±770.23*** | 5.37±3.46*** | 61.13 |
| | 25 | 6/6 | 258.63 ±104.26 | 1230.76 ±635.23* | 3.98±2.78* | 42.67 |
| | 50 | 6/6 | 279.56 ±156.70 | 630.69 ±458.86 | 3.64±1.65 | 20.23 |
| Isovaleryl spiramycin II | 12.5 | 6/6 | 134.33 ±57.77 | 1029.72 ±818.01* | 7.90±6.97* | 70.48 |
| | 25 | 6/6 | 195.76 ±76.95 | 1094.82±687.01* | 6.88±5.66* | 51.42 |
| | 50 | 6/6 | 143.85 ±16.25 | 577.98 ±411.47* | 6.90±7.37* | 36.95 |
| Isovaleryl spiramycin III | 12.5 | 6/6 | 186.22 ±80.26 | 1326.06 ±96.10 ** | 5.52±4.03** | 65.48 |
| | 25 | 6/6 | 274.12 ±83.50 | 1473.96 ±798.75 ** | 3.77±6.51 ** | 49.44 |
| | 50 | 6/6 | 295.39 ±188.69 | 974.67 ±839.14 | 3.66±1.35 | 30.34 |
| *p<0.05 compared with the model group; **p<0.01 compared with the model group, ****p<0.001 compared with the model group | | | | | | |

**5. Inhibition of isovaleryl spiramycin I, II and III on human prostate cancer in nude mice model**

**Establishment of a mouse solid tumor model**

PC3 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = V/Vo (Vo is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 12, and Table 13).

The tumor growth inhibition rates of isovaleryl spiramycin I groups with low, medium and high doses are 33.87%, 51.33% and 71.01%, respectively.

The tumor growth inhibition rates of isovaleryl spiramycin II groups with low, medium and high doses are 23.49%, 40.16% and 50.44%, respectively.

The tumor growth inhibition rates of isovaleryl spiramycin III groups with low, medium and high doses are 25.44%, 40.16% and 60.37%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are 70.43%, 49.14% and 30.72%, respectively.

The relative tumor proliferation rates of isovaleryl spiramycin II groups with low, medium and high doses are 83.04%, 60.08% and 44.48%, respectively.

The relative tumor proliferation rates of isovaleryl spiramycin III groups with low, medium and high doses are 75.75%, 55.02% and 34.57%, respectively.

There are no significant changes in the mouse weight of isovaleryl spiramycin I, II and III groups with low, medium and high doses compared with the model group.

Table 12. Effect of isovaleryl spiramycin I, II and III on the inhibition rate of transplanted tumor of human prostate cancer cell PC3 in nude mice (x±s)

| Group | Dose (mg/kg) | Number of Animals (Start/End) | Body Weight (g) (dl) | Body Weight (g) (d30) | Tumor Weight (g) | Inhibition Rate (%) |
|---|---|---|---|---|---|---|
| Model Group | 0 | 6/6 | 21.3±1.28 | 22.27±1.95 | 1.88±0.75 | -- |
| Cyclophos phamide | 30 | 6/6 | 21.57±0.75 | 16.43±0.78 | 0.56±0.17** | 70.03 |
| Isovaleryl spiramycin I | 25 | 6/6 | 21.17±0.92 | 22.8±0.93 | 1.24±0.51 | 33.87 |
| | 50 | 6/6 | 21.63±0.98 | 22.96±0.60 | 0.91±0.33* | 51.33 |
| | 100 | 6/6 | 20.92±0.58 | 21.97±0.85 | 0.55±0.21** | 71.01 |
| Isovaleryl spiramycin II | 25 | 6/6 | 21.78±0.58 | 21.93±0.85 | 1.44±0.56 | 23.49 |
| | 50 | 6/6 | 20.98±0.8 | 21.68±0.41 | 1.13±0.43 | 40.16 |
| | 100 | 6/6 | 21.37±0.88 | 21.52±0.60 | 0.93±0.27* | 50.44 |
| Isovaleryl spiramycin III | 25 | 6/6 | 21.3±0.75 | 21.67±0.73 | 1.40±0.54 | 25.43 |
| | 50 | 6/6 | 21.3±0.76 | 21.65±0.94 | 1.12±0.45* | 40.16 |
| | 100 | 6/6 | 21.4±0.69 | 21.57±0.48 | 0.75±0.25** | 60.37 |
| *p<0.05 compared with the model group, **p<0.01 compared with the model group, ****p<0.001 compared with the model group | | | | | | |

Table 13. Effect of isovaleryl spiramycin I, II and III on the volume change of transplanted tumor of human prostate cancer cell PC3 in nude mice (x±s)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferatio n Rate (T/C) |
|---|---|---|---|---|---|---|
| Model Group | 0 | 6/6 | 36.77±41.07 | 2458.272±93.21 | 19.56±0.65 | -- |
| Cyclophos phamide | 30 | 6/6 | 155.36±34.96 | 834.50±135.35** | 5.73±2.23** | 29.33 |
| Isovaleryl spiramycin I | 25 | 6/6 | 127.59±6.73 | 1758.15±412.68 | 13.78±3.15 | 70.43 |
| | 50 | 6/6 | 186.59±39.40 | 1706.14±347.50 | 9.61±3.15 | 49.14 |
| | 100 | 6/6 | 175.94±12.32 | 1041.69±247.71* | 6.01±1.78** | 30.72 |
| Isovaleryl spiramycin II | 25 | 6/6 | 154.86±4.11 | 2404.85±672.33 | 16.24±6.00 | 83.04 |
| | 50 | 6/6 | 157.25±38.97 | 1729.14±128.57 | 11.75±3.78 | 60.08 |
| | 100 | 6/6 | 131.11±22.05 | 1162.46±495.46 | 8.70±2.61 | 44.48 |
| Isovaleryl spiramycin III | 25 | 6/6 | 146.91±6.70 | 1914.85±729.54 | 14.82±7.55 | 75.75 |
| | 50 | 6/6 | 187.01±50.28 | 1935.79±228.06 | 10.76±2.24 | 55.01 |
| | 100 | 6/6 | 155.11+7.98 | 1051.03+272.23* | 6.76+1.61** | 34.57 |
| *p<0.05 compared with the model group; **p<0.01 compared with the model group, ***p<0.001 compared with the model group | | | | | | |

6. **Inhibition of isovaleryl spiramycin I, II and III on human breast cancer in nude mice model**

**Establishment of a mouse solid tumor model**

MCF-7 cells in a logarithmic growth phase were taken and subjected to trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = V/Vo (Vo is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the

mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 14, and Table 15).

The tumor growth inhibition rates of isovaleryl spiramycin I groups with low, medium and high doses are 31.10%, 51.72% and 70.12%, respectively.

The tumor growth inhibition rates of isovaleryl spiramycin II groups with low, medium and high doses are 20.55%, 41.72% and 56.81%, respectively.

The tumor growth inhibition rates of isovaleryl spiramycin III groups with low, medium and high doses are 26.75%, 39.08% and 61.78%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are 71.92%, 49.05% and 30.80%, respectively.

The relative tumor proliferation rates of isovaleryl spiramycin II groups with low, medium and high doses are 79.23%, 60.58% and 44.44%, respectively.

The relative tumor proliferation rates of isovaleryl spiramycin III groups with low, medium and high doses are 75.03%, 54.92% and 34.91%, respectively.

There are no significant changes in the mouse weight of isovaleryl spiramycin I, II and III groups with low, medium and high doses compared with the model group.

Table 14. Effect of isovaleryl spiramycin I, II and III on the inhibition rate of transplanted tumor of human breast cancer cell MCF-7 in nude mice (x±s)

| Group | Dose (mg/kg) | Number of Animals (Start/End) | Body Weight (g) (dl) | Body Weight (g) (d30) | Tumor Weight (g) | Inhibition Rate (%) |
|---|---|---|---|---|---|---|
| Model Group | 0 | 6/6 | 21.8±0.91 | 21.58±0.84 | 2.72±0.34 | |
| Cyclophos phamide | 30 | 6/6 | 22.03±0.70 | 17.77±0.65 | 0.81±0.25* 8 | 70.18 |
| Isovaleryl spiramycin I | 25 | 6/6 | 21.33+0.79 | 21.73+0.95 | 1.87±0.25 | 31.10 |
| | 50 | 6/6 | 21.22±0.41 | 21.48±0.73 | 1.31±0.50 | 51.72 |
| | 100 | 6/6 | 22.25±0.92 | 21.8±0.78 | 0.81±0.08* * | 70.12 |
| Isovaleryl spiramycin II | 25 | 6/6 | 21.3±0.67 | 21.78±0.68 | 2.16±0.51 | 20.55 |
| | 50 | 6/6 | 21.1±0.53 | 21.37±0.67 | 1.58±0.51 | 41.72 |
| | 100 | 6/6 | 21.93±0.62 | 21.96±0.68 | 1.17±0.47 | 56.81 |
| Isovaleryl spiramycin III | 25 | 6/6 | 20.73±0.97 | 21.21±0.78 | 1.99±0.81 | 26.75 |
| | 50 | 6/6 | 21.33±0.84 | 21.2±0.75 | 1.66±0.66 | 39.09 |
| | 100 | 6/6 | 21.53±0.56 | 21.73±0.69 | 1.03±0.54* | 61.78 |
| *p<0.05 compared with the model group, **p<0.01 compared with the model group, ****p<0.001 compared with the model group | | | | | | |

Table 15. Effect of isovaleryl spiramycin I, II and III on the volume change of transplanted tumor of human breast cancer cell MCF-7 in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferat ion Rate (T/C) |
|---|---|---|---|---|---|---|
| Model Group | 0 | 6/6 | 137.63±12.68 | 2393.99±69.20 | 17.50±1.47 | |
| Cyclophos phamide | 30 | 6/6 | 103.78±12.65 | 532.62±56.27** | 5.17±0.64** | 29.56 |
| Isovaleryl spiramycin I | 25 | 6/6 | 139.97±26.58 | 1634.85±789.61 | 12.59±7.49 | 71.92 |
| | 50 | 6/6 | 146.57±32.97 | 1162.80±394.71 | 8.58±4.41* | 49.05 |
| | 100 | 6/6 | 124.02±15.14 | 656.95±49.58** | 5.39±0.97** | 30.80 |
| Isovaleryl spiramycin II | 25 | 6/6 | 104.64±10.69 | 1450.36±218.20 | 13.87±1.53 | 79.23 |
| | 50 | 6/6 | 105.81±11.20 | 1113.42±71.14 | 10.60±1.10 | 60.58 |
| | 100 | 6/6 | 119.69±14.63 | 914.69±130.26* | 7.78±1.71* | 44.45 |
| Isovaleryl spiramycin III | 25 | 6/6 | 137.99±20.99 | 1758.49±129.06 | 13.13±3.15 | 75.03 |
| | 50 | 6/6 | 109.95±13.42 | 1043.33±113.01 | 9.61±1.66 | 54.92 |
| | 100 | 6/6 | 113.14±9.02 | 684.21±115.00** | 5.17±0.64** | 34.90 |
| *p<0.05 compared with the model group; **p<0.01 compared with the model group, ***p<0.001 compared with the model group | | | | | | |

**7. Inhibition of isovaleryl spiramycin I, II and III on human breast cancer in nude mice model**

**Establishment of a mouse solid tumor model**

MDA-MB-231 cells in a logarithmic growth phase were taken and subjected to a trypan blue exclusion experiment showing that the cell viability was more than95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the

mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 16, and Table 17).

The tumor growth inhibition rates of isovaleryl spiramycin I groups with low, medium and high doses are 22.84%, 55.17% and 69.11%, respectively.

The tumor growth inhibition rates of isovaleryl spiramycin II groups with low, medium and high doses are 18.17%, 29.66% and 58.91%, respectively.

The tumor growth inhibition rates of isovaleryl spiramycin III groups with low, medium and high doses are 24.35%, 21.01% and 62.93%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are 69.71%, 44.18% and 27.74%, respectively.

The relative tumor proliferation rates of isovaleryl spiramycin II groups with low, medium and high doses are 80.22%, 58.78% and 30.89%, respectively.

The relative tumor proliferation rates of isovaleryl spiramycin III groups with low, medium and high doses were 68.36%, 50.12% and 35.27%, respectively.

There are no significant changes in the mouse weight of isovaleryl spiramycin I, II and III groups with low, medium and high doses compared with the model group.

Table 16. Effect of isovaleryl spiramycin I, II and III on the inhibition rate of transplanted tumor of human breast cancer cell MDA-MB-231 in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.25±1.34 | 24.60±1.71 | 2.32±0.22 | -- |
| Cyclophos phamide | 30 | 6/6 | 21.65±1.50 | 19.26±1.49 | 0.38±0.27*** | 70.54 |
| Isovaleryl spiramycin I | 25 | 6/6 | 21.97±1.25 | 24.25±1.39 | 1.79±0.34* | 22.84 |
| | 50 | 6/6 | 21.24±1.40 | 25.56±1.69 | 1.04±0.44*** | 55.17 |
| | 100 | 6/6 | 21.87±1.49 | 24.47±1.85 | 0.72±0.48*** | 69.11 |
| Isovaleryl spiramycin II | 25 | 6/6 | 20.24±1.89 | 21.32±1.85 | 1.90±0.31* | 18.17 |
| | 50 | 6/6 | 20.02±1.78 | 22.92±1.54 | 1.63±0.32** | 29.66 |
| | 100 | 6/6 | 21.91±1.97 | 22.42±1.41 | 0.95±0.42*** | 58.91 |
| Isovaleryl spiramycin III | 25 | 6/6 | 21.69±1.78 | 23.97±1.81 | 1.76±0.20** | 24.35 |
| | 50 | 6/6 | 21.17±1.6 | 24.36±1.20 | 1.11±0.54** | 52.01 |
| | 100 | 6/6 | 20.43±2.77 | 23.53±1.58 | 0.86±0.44*** | 62.93 |
| *p<0.05 compared with the model group, **p<0.01 compared with the model group, ***p<0.001 compared with the model group | | | | | | |

Table 17. Effect of isovaleryl spiramycin I, II and III on the volume change of transplanted tumor of human breast cancer cell MDA-MB-231 in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferat ion Rate (T/C) |
|---|---|---|---|---|---|---|
| Model Group | 0 | 6/6 | 121.07±9.76 | 1875.22±104.10 | 15.62±2.08 | -- |
| Cyclophos phamide | 30 | 6/6 | 116.90±7.18 | 691.84±337.21*** | 5.89±2.84*** | 37.73 |
| Isovaleryl spiramycin I Isovaleryl spiramycin II | 25 | 6/6 | 111.07±8.47 | 1213.38±299.29** | 10.89±2.52* | 69.71 |
| | 50 | 6/6 | 116.00±8.13 | 807.45±319.26*** | 6.90±2.43*** | 44.18 |
| | 100 | 6/6 | 111.91±10.26 | 458.55±338.74*** | 4.33±3.47*** | 27.74 |
| | 25 | 6/6 | 119.72±6.52 | 1493.96±171.49** | 12.53±1.78* | 80.22 |
| | 50 | 6/6 | 119.07±3.19 | 1094.89±291.37*** | 9.18±2.38*** | 58.78 |
| | 100 | 6/6 | 115.89±9.01 | 700.44±192.38*** | 6.08±1.74*** | 30.89 |
| Isovaleryl spiramycin III | 25 | 6/6 | 114.53±6.52 | 1217.28±267.06** | 10.68±2.47** | 68.36 |
| | 50 | 6/6 | 112.00±10.40 | 860.48±286.44*** | 7.83±2.97*** | 50.12 |
| | 100 | 6/6 | 115.76±7.04 | 647.02±338.62*** | 5.51±2.72*** | 35.27 |
| *p<0.05 compared with the model group; **p<0.01 compared with the model group, ***p<0.001 compared with the model group | | | | | | |

**8. Inhibition of isovaleryl spiramycin I, II and III on human pancreatic cancer in nude mice model**

**Establishment of a mouse solid tumor model**

PANC-1 cells in a logarithmic growth phase were taken and subjected to a trypan blue exclusion experiment showing that the cell viability was more than95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation

rate (T/C) are calculated respectively according to the following formulas, wherein $V=a\times b^2/2$; $RTV = V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 18, and Table 19).

The tumor growth inhibition rates of isovaleryl spiramycin I groups with low, medium and high doses are 31.10%, 51.72% and 70.12%, respectively.

The tumor growth inhibition rates of isovaleryl spiramycin II groups with low, medium and high doses are 20.55%, 41.72% and 56.81%, respectively.

The tumor growth inhibition rates of isovaleryl spiramycin III groups with low, medium and high doses are 26.75%, 39.08% and 61.78%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 71.92%, 49.05% and 30.80%, respectively.

The relative tumor proliferation rates of isovaleryl spiramycin II groups with low, medium and high doses are 79.23%, 60.58% and 44.44%, respectively.

The relative tumor proliferation rates of isovaleryl spiramycin III groups with low, medium and high doses are 75.03%, 54.92% and 34.91%, respectively.

There are no significant changes in the mouse weight of isovaleryl spiramycin I, II and III groups with low, medium and high doses compared with the model group.

Table 18. Effect of isovaleryl spiramycin I, II and III on the inhibition rate of transplanted tumor of human pancreatic cancer cell PANC-1 in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.8±0.91 | 21.58±0.84 | 2.72±0.34 | |
| Cyclophos phamide | 30 | 6/6 | 22.03±0.70 | 17.77±0.65 | 0.81±0.25*8 | 70.18 |
| Isovaleryl spiramycin I | 12.5 | 6/6 | 21.33±0.79 | 21.73±0.95 | 1.87±0.25 | 31.10 |
| | 25 | 6/6 | 21.22±0.41 | 21.48±0.73 | 1.31±0.50 | 51.72 |
| | 50 | 6/6 | 22.25±0.92 | 21.8±0.78 | 0.81±0.08** | 70.12 |
| Isovaleryl spiramycin II | 12.5 | 6/6 | 21.3±0.67 | 21.78±0.68 | 2.16±0.51 | 20.55 |
| | 25 | 6/6 | 21.1±0.53 | 21.37±0.67 | 1.58±0.51 | 41.72 |
| | 50 | 6/6 | 21.93±0.62 | 21.96±0.68 | 1,17±0.47 | 56.81 |
| Isovaleryl spiramycin III | 12.5 | 6/6 | 20.73±0.97 | 21.21±0.78 | 1.99±0.81 | 26.75 |
| | 25 | 6/6 | 21.33±0.84 | 21.2±0.75 | 1.66±0.66 | 39.09 |
| | 50 | 6/6 | 21.53±0.56 | 21.73±0.69 | 1.03±0.54* | 61.78 |

*p<0.05 compared with the model group, **p<0.01 compared with the model group, ***p<0.001 compared with the model group

Table 19. Effect of isovaleryl spiramycin I, II and III on the volume change of transplanted tumor of human pancreatic cancer cell PANC-1 in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferat ion Rate (T/C) |
|---|---|---|---|---|---|---|
| Model Group | 0 | 6/6 | 137.63±12.68 | 2393.99±69.20 | 17.50±1.47 | |
| Cyclophos phamide | 30 | 6/6 | 103.78±12.65 | 532.62±56.27** | 5.17±0.64** | 29.56 |
| Isovaleryl spiramycin I | 12.5 | 6/6 | 139.97±26.58 | 1634.85±789.61 | 12.59±7.49 | 71.92 |
| | 25 | 6/6 | 146.57±32.97 | 1162.80±394.71** | 8.58±4.41* | 49.05 |
| | 50 | 6/6 | 124.02±15.14 | 656.95±49.58*** | 5.39±0.97** | 30.80 |
| Isovaleryl spiramycin II | 12.5 | 6/6 | 104.64±10.69 | 1450.36±218.20 | 13.87±1.53 | 79.23 |
| | 25 | 6/6 | 105.81±11.20 | 1113.42±71.14 | 10.60±1.10 | 60.58 |
| | 50 | 6/6 | 119.69±14.63 | 914.69±130.26** | 7.78±1.71* | 44.45 |
| Isovaleryl spiramycin III | 12.5 | 6/6 | 137.99±20.99 | 1758.49±129.06 | 13.13±3.15 | 75.03 |
| | 25 | 6/6 | 109.95±13.42 | 1043.33±113.01 | 9.61±1.66 | 54.92 |
| | 50 | 6/6 | 113.14±9.02 | 684.21±115.00** | 5.17±0.64** | 34.90 |
| *p<0.05 compared with the model group; **p<0.01 compared with the model group, ***p<0.001 compared with the model group | | | | | | |

**9. Inhibition of isovaleryl spiramycin I, II and III on human hepatoma in nude mice model**

**Establishment of a mouse solid tumor model**

HepG-2 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) $\times$ 100%.

The test results show that compared with the control group, each drug-administered group has a certain degree of inhibition on the tumor growth inhibition rate, tumor volume, relative tumor volume and relative tumor proliferation rate (see Table 20, and Table 21).

The tumor growth inhibition rates of isovaleryl spiramycin I groups with low, medium and high doses are 24.43%, 57.93% and 68.22%, respectively.

The tumor growth inhibition rates of isovaleryl spiramycin II groups with low, medium and high doses are 21.07%, 31.43% and 61.56%, respectively.

The tumor growth inhibition rates of isovaleryl spiramycin III groups with low, medium and high doses of are 38.92%, 60.54% and 63.28%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 65.03%, 42.12% and 27.49%, respectively.

The relative tumor proliferation rates of isovaleryl spiramycin II groups with low, medium and high doses are 81.03%, 57.02% and 39.31%, respectively.

The relative tumor proliferation rates of isovaleryl spiramycin III groups with low, medium and high doses are 64.69%, 43.18% and 32.71%, respectively.

There are no significant changes in the mouse weight of isovaleryl spiramycin I, II and III groups with low, medium and high doses compared with the model group.

Table 20. Effect of isovaleryl spiramycin I, II and III on the inhibition rate of transplanted tumor of human hepatoma cell HepG-2 in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.21±1.42 | 23.75±1.71 | 2.43±0.23 | - |
| Cyclophos phamide | 30 | 6/6 | 21.89±1.84 | 19.67±1.49 | 0.07±0.38*** | 71.04 |
| Isovaleryl spiramycin I | 25 | 6/6 | 21.36±1.67 | 23.24±1.33 | 1.84±0.23** | 24.43 |
| | 50 | 6/6 | 21.47±1.58 | 23.64±1.67 | 1.02±0.45** | 57.93 |
| | 100 | 6/6 | 21.37±1.36 | 22.867±1.57 | 0.77±0.44*** | 68.22 |
| Isovaleryl | 25 | 6/6 | 21.98±1.74 | 23.35±1.83 | 1.92±0.32* | 21.07 |
| spiramycin II | 50 | 6/6 | 20.75±1.86 | 22.22±1.62 | 1.67±0.32** | 31.43 |
| | 100 | 6/6 | 21.48±1.98 | 22.45±1.28 | 0.93±0.41*** | 61.56 |

(continued)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Isovaleryl spiramycin III | 25 | 6/6 | 21.07±1.24 | 23.94±1.67 | 1.48±0.42** | 38.92 |
| | 50 | 6/6 | 21.25±1.86 | 24.36±1.53 | 0.96±0.44*** | 60.54 |
| | 100 | 6/6 | 20.47±2.24 | 23.64±1.44 | 0.89±0.46*** | 63.28 |
| *p<0.05 compared with the model group, **p<0.01 compared with the model group, ***p<0.001 compared with the model group | | | | | | |

Table 21. Effect of isovaleryl spiramycin I, II and III on the volume change of transplanted tumor of human hepatoma cell HepG-2 in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Prolifera tion Rate (T/C) |
|---|---|---|---|---|---|---|
| Model Group | 0 | 6/6 | 117.98±9.34 | 1960.30±92.59 | 16.74±2.02 | - |
| Cyclophos phamide | 30 | 6/6 | 119.66±7.65 | 792.56±287.31*** | 6.58±2.18*** | 39.27 |
| Isovaleryl spiramycin I | 25 | 6/6 | 114.02±11.68 | 1248.33±337.59** | 10.89±2.67** | 65.03 |
| | 50 | 6/6 | 122.31±4.68 | 851.37±371.80*** | 7.05±3.35*** | 42.12 |
| | 100 | 6/6 | 110.06±9.58 | 481.16±326.94*** | 4.60±3.39*** | 27.49 |
| Isovaleryl spiramycin II | 25 | 6/6 | 122.82±4.10 | 1666.11±202.07* | 13.57±1.64* | 81.03 |
| | 50 | 6/6 | 118.60±4.30 | 1127.90±319.12** | 9.55±2.84** | 57.02 |
| | 100 | 6/6 | 118.70±7.20 | 783.73±312.41** | 6.58±2.48** | 39.31 |
| Isovaleryl spiramycin III | 25 | 6/6 | 116.67±8.32 | 1261.48±283.95** | 10.83±2.47** | 64.69 |
| | 50 | 6/6 | 119.43±10.86 | 852.87±293.29*** | 7.23±2.57*** | 43.18 |
| | 100 | 6/6 | 118.26±6.57 | 641.99±364.25*** | 5.48±3.19*** | 32.71 |
| *p<0.05 compared with the model group; **p<0.01 compared with the model group, ***p<0.001 compared with the model group | | | | | | |

**10. Inhibition of isovaleryl spiramycin I, II and III on human non-small cell lung cancer in nude mice model**

**Establishment of a mouse solid tumor model**

A549 cells in a logarithmic growth phase were taken and subjected to a trypan blue exclusion experiment

showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV$\times$100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) $\times$ 100%.

The test results show that compared with the control group, each drug-administered group has a certain degree of inhibition on the tumor growth inhibition rate, tumor volume, relative tumor volume and relative tumor proliferation rate (see Table 22, and Table 23).

The tumor growth inhibition rates of isovaleryl spiramycin I groups with low, medium and high doses are 24.00%, 58.10% and 69.52%, respectively.

The tumor growth inhibition rates of isovaleryl spiramycin II groups with low, medium and high doses are 20.73%, 31.87% and 60.19%, respectively.

The tumor growth inhibition rates of isovaleryl spiramycin III groups with low, medium and high doses are 37.99%, 55.95% and 66.53%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 67.18%, 41.93% and 28.35%, respectively.

The relative tumor proliferation rates of isovaleryl spiramycin II groups with low, medium and high doses are 83.41%, 58.75% and 39.42%, respectively.

The relative tumor proliferation rates of isovaleryl spiramycin III groups with low, medium and high doses are 65.93%, 47.25% and 33.04%, respectively.

There are no significant changes in the mouse weight of isovaleryl spiramycin I, II and III groups with low, medium and high doses compared with the model group.

Table 22. Effect of isovaleryl spiramycin I, II and III on the inhibition rate of transplanted tumor of human non-small cell lung cancer cell A549 in nude mice ($\bar{x}\pm s$)

| Group | Dose (mg/kg) | Number of Animals (Start/End) | BodyWeight(g) (d1) | Body Weight (g) (d30) | Tumor Weight (g) | Inhibition Rate (%) |
|---|---|---|---|---|---|---|
| Model Group | 0 | 6/6 | 21.21±1.34 | 24.57±1.71 | 2.39±0.24 | -- |
| Cyclophos phamide | 30 | 6/6 | 21.89±1.22 | 19.27±1.49 | 0.70±0.39*** | 70.59 |

(continued)

| Group | Dose (mg/kg) | Number of Animals (Start/End) | BodyWeight(g) (d1) | Body Weight (g) (d30) | Tumor Weight (g) | Inhibition Rate (%) |
|---|---|---|---|---|---|---|
| Isovaleryl spiramycin I | 25 | 6/6 | 21.54±1.27 | 24.20±1.33 | 1.82±0.19** | 24.00 |
| | 50 | 6/6 | 21.48±1.46 | 25.56±1.67 | 1.00±0.44** | 58.10 |
| | 100 | 6/6 | 21.36±1.82 | 24.47±1.98 | 0.73±0.45** | 69.52 |
| Isovaleryl spiramycin II | 25 | 6/6 | 20.91±1.07 | 21.32±1.88 | 1.90±0.31* | 20.73 |
| | 50 | 6/6 | 20.03±1.88 | 12.92±1.68 | 1.63±0.32** | 31.87 |
| | 100 | 6/6 | 21.71±1.94 | 12.42±1.37 | 0.95±0.42*** | 60.19 |
| Isovaleryl spiramycin III | 25 | 6/6 | 21.68±1.25 | 23.97±1.01 | 1.49±0.42* | 37.99 |
| | 50 | 6/6 | 21.99±1.40 | 24.36±1.73 | 1.06±0.49*** | 55.95 |
| | 100 | 6/6 | 20.57±2.28 | 23.53±1.63 | 0.80±0.43*** | 66.53 |
| *p<0.05 compared with the model group, **p<0.01 compared with the model group, ***p<0.001 compared with the model group | | | | | | |

Table 23. Effect of isovaleryl spiramycin I, II and III on the volume change of transplanted tumor of human non-small cell lung cancer cell A549 in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferati on Rate (T/C) |
|---|---|---|---|---|---|---|
| Model Group | 0 | 6/6 | 118.12±9.47 | 1901.00±131.19 | 16.23±2.25 | -- |
| Cyclophos phamide | 30 | 6/6 | 115.15±10.00 | 733.89±352.79*** | 9.47±2.91** | 39.73 |
| Isovaleryl spiramycin I | 25 | 6/6 | 111.89±12.17 | 1227.24±329.84** | 10.91±2.63** | 67.18 |
| | 50 | 6/6 | 119.73±5.73 | 750.44±220.77*** | 6.40±1.75*** | 39.42 |
| | 100 | 6/6 | 110.27±11.43 | 475.83±355.37*** | 4.60±3.69*** | 28.35 |
| Isovaleryl spiramycin II | 25 | 6/6 | 119.67±7.25 | 1614.91±65.26** | 13.54±1.07* | 83.41 |
| | 50 | 6/6 | 117.96±4.88 | 1129.10±326.54** | 9.54±2.62*** | 58.75 |
| | 100 | 6/6 | 116.96±6.56 | 917.10±270.86*** | 7.78±2.03*** | 47.94 |
| Isovaleryl spiramycin III | 25 | 6/6 | 113.96±6.85 | 1216.86±295.20** | 10.70±2.64** | 65.93 |
| | 50 | 6/6 | 113.65±11.57 | 856.65±281.96*** | 7.67±2.86*** | 47.25 |
| | 100 | 6/6 | 118.69±8.13 | 632.73±350.89*** | 5.36±3.01*** | 33.04 |
| *p<0.05 compared with the model group; **p<0.01 compared with the model group, ***p<0.001 compared with the model group | | | | | | |

## 11. Inhibition of isovaleryl spiramycin I, II and III on human glioma in nude mice model

### Establishment of a mouse solid tumor model

U251 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

### Calculation of tumor volume and relative tumor proliferation rate

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV$\times$100%.

### Calculation of tumor growth inhibition rate

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) $\times$ 100%.

The test results show that compared with the control group, each drug-administered group has a certain degree of inhibition on the tumor growth inhibition rate, tumor volume, relative tumor volume and relative tumor proliferation rate (see Table 24, and Table 25).

The tumor growth inhibition rates of isovaleryl spiramycin I groups with low, medium and high doses are 30.94%, 44.53% and 69.21%, respectively.

The tumor growth inhibition rates of isovaleryl spiramycin II groups with low, medium and high doses are 10.91%, 15.81% and 40.26%, respectively.

The tumor growth inhibition rates of isovaleryl spiramycin III groups with low, medium and high doses are 15.45%, 32.74% and 59.56%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 76.40%, 44.54% and 25.80%, respectively.

The relative tumor proliferation rates of isovaleryl spiramycin II groups with low, medium and high doses are 76.14%, 51.88% and 43.26%, respectively.

The relative tumor proliferation rates of isovaleryl spiramycin III groups with low, medium and high doses are 68.16%, 54.34% and 41.10%, respectively.

There are no significant changes in the mouse weight of isovaleryl spiramycin I, II and III groups with low, medium and high doses compared with the model group.

Table 24. Effect of isovaleryl spiramycin I, II and III on the inhibition rate of transplanted tumor of human glioma cell U251 cells in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.02±0.37 | 20.50±0.25 | 1.38±0.05 | -- |

(continued)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Cyclophos phamide | 30 | 6/6 | 20.72±0.48 | 19.40±1.40 | 0.42±0.06*** | 69.31 |
| Isovaleryl spiramycin I | 25 | 6/6 | 21.03±0.55 | 21.09±0.53 | 0.96±0.08* | 30.94 |
| | 50 | 6/6 | 20.78±0.66 | 21.08±0.54 | 0.77±0.09** | 44.53 |
| | 100 | 6/6 | 21.10±0.35 | 20.86±0.66 | 0.43±0.01** | 69.21 |
| Isovaleryl spiramycin II | 25 | 6/6 | 20.77±0.65 | 20.85±0.79 | 1.23±0.12 | 10.91 |
| | 50 | 6/6 | 21.25±0.46 | 20.86±0.43 | 1.16±0.10* | 15.81 |
| | 100 | 6/6 | 20.92±0.82 | 21.47±0.70 | 0.83±0.12*** | 40.26 |
| Isovaleryl spiramycin III | 25 | 6/6 | 20.96±0.58 | 20.70±0.51 | 1.17±0.11** | 15.45 |
| | 50 | 6/6 | 21.25±0.53 | 21.10±0.60 | 0.93±0.12** | 32.74 |
| | 100 | 6/6 | 21.33±0.62 | 21.03±0.55 | 0.56±0.05*** | 59.56 |
| *p<0.05 compared with the model group, **p<0.01 compared with the model group, ***p<0.001 compared with the model group | | | | | | |

Table 25. Effect of isovaleryl spiramycin I, II and III on the volume change of transplanted tumor of human glioma cell U251 cells in nude mice ($\bar{x}\pm s$)

| Group | Dose (mg/kg) | Number of Animals (Start/En d) | Tumor Volume (mm3) (d1) | Tumor Volume (mm3) (d30) | Relative Tumor Volume (RTV) | Relative Tumor Prolifera tion Rate (T/C) |
|---|---|---|---|---|---|---|
| Model Group | 0 | 6/6 | 125.74±10.43 | 2082.09±83.17 | 16.69±2.03 | -- |
| Cyclophos phamide | 30 | 6/6 | 119.00±11.13 | 504.56±93.91** | 4.23±0.95*** | 62.92 |
| Isovaleryl spiramycin I | 25 | 6/6 | 122.81±10.35 | 1550.33±72.99** | 12.69±1.08** | 76.40 |
| | 50 | 6/6 | 124.39±10.31 | 930.42±204.72** | 10.67±1.09*** | 44.54 |
| | 100 | 6/6 | 124.88±6.57 | 537.02±93.66*** | 10.05±0.71*** | 25.80 |
| Isovaleryl spiramycin II | 25 | 6/6 | 125.90±11.45 | 1596.45±267.65* | 14.73±1.33* | 76.14 |
| | 50 | 6/6 | 118.33±11.65 | 1030.10±207.19** | 14.31±0.98* | 51.88 |
| | 100 | 6/6 | 122.74±10.75 | 890.78±225.34** | 12.88±1.54** | 43.26 |
| Isovaleryl spiramycin III | 25 | 6/6 | 124.24±10.18 | 1413.41±221.90* | 13.47±1.67** | 68.16 |
| | 50 | 6/6 | 121.24±9.15 | 1096.57±173.49** | 12.63±1.27*** | 54.34 |
| | 100 | 6/6 | 120.86±6.34 | 832.79±182.98** | 10.96±0.82*** | 41.10 |
| *p<0.05 compared with the model group; **p<0.01 compared with the model group, ***p<0.001 compared with the model group | | | | | | |

## 12. Inhibition of isovaleryl spiramycin I, II and III on human glioblastoma in nude mice model

### Establishment of a mouse solid tumor model

A172 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a temozolomide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

### Calculation of tumor volume and relative tumor proliferation rate

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

### Calculation of tumor growth inhibition rate

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 26, and Table 27).

The tumor growth inhibition rates of isovaleryl spiramycin I groups with low, medium and high doses are 30.75%, 44.26% and 68.79%, respectively.

The tumor growth inhibition rates of isovaleryl spiramycin II groups with low, medium and high doses are 10.85%, 15.71% and 40.01%, respectively.

The tumor growth inhibition rates of isovaleryl spiramycin III groups with low, medium and high doses are 15.35%, 32.54% and 59.19%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 74.49%, 43.43% and 25.16%, respectively.

The relative tumor proliferation rates of isovaleryl spiramycin II groups with low, medium and high doses are 74.24%, 50.59% and 42.18%, respectively.

The relative tumor proliferation rates of isovaleryl spiramycin III groups with low, medium and high doses are 66.45%, 52.89% and 40.08%, respectively.

There are no significant changes in the mouse weight of isovaleryl spiramycin I, II and III groups with low, medium and high doses compared with the model group.

Table 26. Effect of isovaleryl spiramycin I, II and III on the inhibition rate of transplanted tumor of human glioblastoma cell A172 in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 20.80±0.66 | 20.50±0.75 | 1.39±0.05 | -- |

(continued)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Temozolomide | 50 | 6/6 | 21.07±0.57 | 19.83±1.24 | 0.43±0.06*** | 68.89 |
| Isovaleryl spiramycin I | 12.5 | 6/6 | 21.06±0.43 | 20.34±0.17 | 0.96±0.08** | 30.75 |
| | 25 | 6/6 | 20.98±0.73 | 20.93±0.54 | 0.77±0.09** | 44.26 |
| | 50 | 6/6 | 21.00±0.65 | 20.88±0.58 | 0.43±0.07*** | 68.79 |
| Isovaleryl spiramycin II | 12.5 | 6/6 | 21.36±0.67 | 21.36±0.87 | 1.24±0.12 | 10.85 |
| | 25 | 6/6 | 21.10±0.62 | 21.30±0.89 | 1.17±0.10* | 15.71 |
| | 50 | 6/6 | 21.08±0.74 | 20.97±0.68 | 0.84±0.12** | 40.01 |
| Isovaleryl spiramycin III | 12.5 | 6/6 | 21.54±0.36 | 20.64±0.54 | 1.18±0.11* | 15.35 |
| | 25 | 6/6 | 20.98±0.47 | 21.54±0.72 | 0.94±0.12** | 32.54 |
| | 50 | 6/6 | 21.59±0.32 | 20.67±0.56 | 0.57±0.05*** | 59.19 |
| *p<0.05 compared with the model group, **p<0.01 compared with the model group, ***p<0.001 compared with the model group | | | | | | |

Table 27. Effect of isovaleryl spiramycin I, II and III on the volume change of transplanted tumor of human glioblastoma cell A172 in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferation Rate (T/C) |
|---|---|---|---|---|---|---|
| Model Group | 0 | 6/6 | 123.2±10.22 | 2040.44±81.50 | 16.69±2.03 | --- |
| Temozolomide | 50 | 6/6 | 116.6±10.91 | 482.11±89.74*** | 4.12±2.03*** | 61.35 |
| Isovaleryl spiramycin I | 12.5 | 6/6 | 120.36±10.15 | 1519.32±71.53* | 12.69±1.08** | 74.49 |
| | 25 | 6/6 | 121.90±10.10 | 889.01±195.61** | 10.67±1.08*** | 43.43 |
| | 50 | 6/6 | 122.38±6.44 | 513.12±89.49*** | 10.05±0.71*** | 25.16 |
| Isovaleryl spiramycin II | 12.5 | 6/6 | 123.39±11.22 | 1525.41±255.74* | 14.73±1.33* | 74.24 |
| | 25 | 6/6 | 115.96±11.42 | 984.26±197.97** | 14.31±0.98* | 50.59 |
| | 50 | 6/6 | 120.28±10.53 | 851.14±215.31** | 12.88±1.54** | 42.18 |

(continued)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferation Rate (T/C) |
|---|---|---|---|---|---|---|
| Isovaleryl spiramycin III | 12.5 | 6/6 | 121.75±9.98 | 1305.51±212.02* | 13.47±1.67** | 66.45 |
| | 25 | 6/6 | 118.81±8.97 | 1047.77±165.77** | 12.63±1.27*** | 52.89 |
| | 50 | 6/6 | 118.44±6.21 | 795.73±174.84*** | 10.96±0.82*** | 40.08 |
| *p<0.05 compared with the model group; **p<0.01 compared with the model group, ***p<0.001 compared with the model group | | | | | | |

**13. Inhibition of isovaleryl spiramycin I, II and III on human lymphoma in nude mice model**

**Establishment of a mouse solid tumor model**

U937 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 28, and Table 29).

The tumor growth inhibition rates of isovaleryl spiramycin I groups with low, medium and high doses are 41.73%, 50.73% and 65.03%, respectively.

The tumor growth inhibition rates of isovaleryl spiramycin II groups with low, medium and high doses are 25.61%, 35.44% and 43.63%, respectively.

The tumor growth inhibition rates of isovaleryl spiramycin III groups with low, medium and high doses are 41.19%, 53.03% and 61.77%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are

68.65%, 39.74% and 35.25%, respectively.

The relative tumor proliferation rates of isovaleryl spiramycin II groups with low, medium and high doses are 74.19%, 52.10% and 46.09%, respectively.

The relative tumor proliferation rates of isovaleryl spiramycin III groups with low, medium and high doses are 63.36%, 49.30% and 38.66%, respectively.

There are no significant changes in the mouse weight of isovaleryl spiramycin I, II and III groups with low, medium and high doses compared with the model group.

Table 28. Effect of isovaleryl spiramycin I, II and III on the inhibition rate of transplanted tumor of human lymphoma cell U937 in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 20.77±0.51 | 21.11±0.53 | 1.94±0.27 | -- |
| Cyclophos phamide | 30 | 6/6 | 21.00±0.72 | 19.72±0.95 | 0.68±0.12*** | 65.11 |
| Isovaleryl spiramycin I | 12.5 | 6/6 | 21.19±0.66 | 21.16±0.74 | 1.13±0.60* | 41.73 |
| | 25 | 6/6 | 21.28±0.52 | 21.01±0.68 | 0.95±0.42** | 50.73 |
| | 50 | 6/6 | 20.64±0.39 | 20.94±0.77 | 0.68±0.09*** | 65.03 |
| Isovaleryl spiramycin II | 12.5 | 6/6 | 21.10±0.64 | 20.97±0.74 | 1.44±0.64 | 25.61 |
| | 25 | 6/6 | 21.05±0.30 | 21.03±0.632 | 1.25±0.66* | 35.44 |
| | 50 | 6/6 | 20.80±0.51 | 21.22±0.51 | 1.09±0.62* | 43.63 |
| Isovaleryl spiramycin III | 12.5 | 6/6 | 20.84±0.68 | 20.92±0.63 | 1.14±0.54* | 41.19 |
| | 25 | 6/6 | 20.91±0.58 | 20.74±0.352 | 0.91±0.51** | 53.03 |
| | 50 | 6/6 | 21.16±0.61 | 21.24±0.50 | 0.74±0.18*** | 61.77 |
| *p<0.05 compared with the model group, **p<0.01 compared with the model group, ***p<0.001 compared with the model group | | | | | | |

Table 29. Effect of isovaleryl spiramycin I, II and III on the volume change of transplanted tumor of human lymphoma cell U937 in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Prolifer ation Rate (T/C) |
|---|---|---|---|---|---|---|
| Model Group | 0 | 6/6 | 115.70±9.96 | 1986.59±516.10 | 17.04±4.02 | -- |
| Cyclophos phamide | 30 | 6/6 | 125.00±32.61 | 722.72±55.32** | 6.04±1.26*** | 35.44 |
| Isovaleryl spiramycin I | 12.5 | 6/6 | 108.43±6.49 | 1270.43±435.28* | 11.70±3.59* | 68.65 |
| | 25 | 6/6 | 108.61±9.06 | 734.22±76.87** | 6.77±0.57** | 39.74 |
| | 50 | 6/6 | 109.98±5.88 | 660.17±37.49** | 6.01±2.26** | 35.25 |

(continued)

| Group | Dose (mg/kg) | Number of Animals (Start/End) | Tumor Volume (mm3) (d1) | Tumor Volume (mm3) (d30) | Relative Tumor Volume (RTV) | Relative Tumor Prolifer ation Rate (T/C) |
|---|---|---|---|---|---|---|
| Isovaleryl spiramycin II | 12.5 | 6/6 | 111.58±23.26 | 1400.95±558.82 | 12.64±5.00 | 74.19 |
| | 25 | 6/6 | 120.21±24.68 | 1117.59±565.29* | 8.88±2.64** | 52.10 |
| | 50 | 6/6 | 111.50±7.03 | 885.75±418.53** | 7.85±3.27** | 46.09 |
| Isovaleryl spiramycin III | 12.5 | 6/6 | 119.70±18.30 | 1314.25±479.76* | 10.80±3.02* | 63.36 |
| | 25 | 6/6 | 112.93±27.42 | 939.79±296.72** | 8.40±2.25** | 49.30 |
| | 50 | 6/6 | 112.85±21.90 | 728.49±51.66** | 6.59±0.90** | 38.66 |
| *p<0.05 compared with the model group; **p<0.01 compared with the model group, ***p<0.001 compared with the model group | | | | | | |

**14. Inhibition of isovaleryl spiramycin I, II and III on human cervical cancer in nude mice model**

**Establishment of a mouse solid tumor model**

HeLa cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 30, and Table 31).

The tumor growth inhibition rates of isovaleryl spiramycin I groups with low, medium and high doses are 46.69%, 51.57% and 65.55%, respectively.

The tumor growth inhibition rates of isovaleryl spiramycin II groups with low, medium and high doses are 30.67%, 42.90% and 43.30%, respectively.

The tumor growth inhibition rates of isovaleryl spiramycin III groups with low, medium and high doses are 39.33%, 52.41% and 61.68%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are 69.18%, 39.57% and 30.91%, respectively.

The relative tumor proliferation rates of isovaleryl spiramycin II groups with low, medium and high doses are 30.67%, 42.90% and 43.30%, respectively.

The relative tumor proliferation rates of isovaleryl spiramycin III groups with low, medium and high doses are 39.33%, 52.41% and 61.68%, respectively.

There are no significant changes in the mouse weight of isovaleryl spiramycin I, II and III groups with low, medium and high doses compared with the model group.

Table 30. Effect of isovaleryl spiramycin I, II and III on the inhibition rate of transplanted tumor of human cervical cancer cell HeLa in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.33±0.48 | 21.27±0.68 | 1.93±0.21 | -- |
| Cyclophos phamide | 30 | 6/6 | 21.10±0.69 | 19.73±1.22 | 0.66±0.13*** | 65.74 |
| Isovaleryl spiramycin I | 12.5 | 6/6 | 20.72±0.49 | 21.10±0.68 | 1.03±0.59* | 46.69 |
| | 25 | 6/6 | 21.07±0.44 | 21.23±0.73 | 0.94±0.48** | 51.57 |
| | 50 | 6/6 | 20.82±0.72 | 20.95±0.45 | 0.67±0.09*** | 65.55 |
| Isovaleryl spiramycin II | 12.5 | 6/6 | 20.92±0.46 | 21.16±0.51 | 1.34±0.60 | 30.67 |
| | 25 | 6/6 | 20.87±0.34 | 21.25±0.55 | 1.10±0.62* | 42.90 |
| | 50 | 6/6 | 20.77±0.43 | 21.09±0.36 | 1.10±0.61* | 43.30 |
| Isovaleryl spiramycin III | 12.5 | 6/6 | 21.30±0.64 | 20.67±0.45 | 1.17±0.64* | 39.33 |
| | 25 | 6/6 | 20.87±0.78 | 20.80±0.55 | 0.92±0.49** | 52.41 |
| | 50 | 6/6 | 21.09±0.51 | 21.10±0.52 | 0.75±0.11*** | 61.68 |
| *p<0.05 compared with the model group, **p<0.01 compared with the model group, ***p<0.001 compared with the model group | | | | | | |

Table 31. Effect of isovaleryl spiramycin I, II and III on the volume change of transplanted tumor of human cervical cancer cell HeLa in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferati on Rate (T/C) |
|---|---|---|---|---|---|---|
| Model Group | 0 | 6/6 | 119.20±11.23 | 1991.26±516.28 | 16.58±3.86 | -- |
| Cyclophos phamide | 30 | 6/6 | 127.17±35.82 | 724.89±53.90** | 6.00±1.35*** | 36.17 |

(continued)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferati on Rate (T/C) |
|---|---|---|---|---|---|---|
| Isovaleryl spiramycin I Isovaleryl spiramycin II | 12.5 | 6/6 | 112.43±6.87 | 1287.77±552.57* | 11.47±4.97 | 69.18 |
| | 25 | 6/6 | 112.78±9.72 | 738.39±75.32** | 6.56±0.55** | 39.57 |
| | 50 | 6/6 | 114.15±6.08 | 588.18±73.34** | 5.13±0.68*** | 30.91 |
| | 12.5 | 6/6 | 115.41±23.27 | 1301.28±391.77* | 11.36±2.90 | 68.55 |
| | 25 | 6/6 | 125.04±20.71 | 1184.42±369.49* | 9.30±1.74** | 56.07 |
| | 50 | 6/6 | 111.84±4.83 | 851.58±93.66** | 7.62±0.85** | 45.96 |
| Isovaleryl spiramycin III | 12.5 | 6/6 | 123.36±21.45 | 1281.92±242.71* | 1.43±1.47** | 62.92 |
| | 25 | 6/6 | 115.2±27.18 | 1007.46±481.36** | 8.63±3.37** | 52.05 |
| | 50 | 6/6 | 113.35±20.84 | 727.16±46.92** | 6.52±0.73** | 39.33 |
| *p<0.05 compared with the model group; **p<0.01 compared with the model group, ***p<0.001 compared with the model group | | | | | | |

**15. Inhibition of isovaleryl spiramycin I, II and III on human renal clear cell adenocarcinoma in nude mice model**

**Establishment of a mouse solid tumor model**

786-O cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor

growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 32, and Table 33).

The tumor growth inhibition rates of isovaleryl spiramycin I groups with low, medium and high doses are 34.70%, 39.22% and 64.36%, respectively.

The tumor growth inhibition rates of isovaleryl spiramycin II groups with low, medium and high doses are 19.69%, 41.09% and 60.00%, respectively.

The tumor growth inhibition rates of isovaleryl spiramycin III groups with low, medium and high doses are 35.80%, 52.14% and 62.49%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are 37.88%, 37.19% and 36.89%, respectively.

The relative tumor proliferation rates of isovaleryl spiramycin II groups with low, medium and high doses are 76.92%, 53.61% and 35.74%, respectively.

The relative tumor proliferation rates of isovaleryl spiramycin III groups with low, medium and high doses are 73.13%, 51.33% and 34.20%, respectively.

There are no significant changes in the mouse weight of isovaleryl spiramycin I, II and III groups with low, medium and high doses compared with the model group.

Table 32. Effect of isovaleryl spiramycin I, II and III on the inhibition rate of transplanted tumor of human renal clear cell adenocarcinoma cell 786-O in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.83±1.32 | 24.17±1.29 | 2.14±0.07 | -- |
| Cyclophos phamide | 30 | 6/6 | 21.58±0.81 | 19.42±0.67 | 0.63±0.04*** | 70.51 |
| Isovaleryl spiramycin I Isovaleryl spiramycin II | 25 | 6/6 | 21.87±0.89 | 22.95±0.88 | 1.40±0.40** | 34.70 |
| | 50 | 6/6 | 22.00±0.94 | 22.82±0.64 | 1.31±0.35*** | 39.22 |
| | 100 | 6/6 | 21.88±0.54 | 23.00±0.67 | 0.76±0.13*** | 64.36 |
| | 25 | 6/6 | 22.05±0.71 | 23.33±0.54 | 1.72±0.27** | 19.69 |
| | 50 | 6/6 | 22.42±0.54 | 23.42±0.50 | 1.26±0.31** | 41.09 |
| | 100 | 6/6 | 22.33±0.41 | 23.45±0.91 | 0.85±0.16*** | 60.00 |
| Isovaleryl spiramycin III | 25 | 6/6 | 22.72±0.78 | 23.85±0.89 | 1.38±0.42** | 35.80 |
| | 50 | 6/6 | 21.43±1.08 | 22.33±1.09 | 1.02±0.27*** | 52.14 |
| | 100 | 6/6 | 22.28±1.72 | 23.43±1.69 | 0.80±0.05*** | 62.49 |
| *p<0.05 compared with the model group, **p<0.01 compared with the model group, ***p<0.001 compared with the model group | | | | | | |

Table 33. Effect of isovaleryl spiramycin I, II and III on the volume change of transplanted tumor of human renal clear cell adenocarcinoma cell 786-O in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferat ion Rate (T/C) |
|---|---|---|---|---|---|---|
| Model Group | 0 | 6/6 | 114.09±9.07 | 2060.98±168.33 | 18.06±1.78 | -- |
| Cyclophos phamide | 30 | 6/6 | 108.64±14.40 | 734.23±24.66*** | 6.76±0.79*** | 37.41 |
| Isovaleryl spiramycin I | 25 | 6/6 | 96.85±9.98 | 677.21±58.76*** | 6.99±0.83*** | 37.88 |
| | 50 | 6/6 | 96.82±8.52 | 664.44±73.49*** | 6.86±1.01*** | 37.19 |
| | 100 | 6/6 | 103.25±9.71 | 702.74±37.66*** | 6.81±0.31*** | 36.87 |
| Isovaleryl spiramycin II | 25 | 6/6 | 96.82±8.52 | 1415.80±201.11* | 14.62±1.93* | 76.92 |
| | 50 | 6/6 | 96.82±8.52 | 982.68±265.02* | 10.19±2.62** | 53.61 |
| | 100 | 6/6 | 103.26±9.71 | 701.57±127.91** | 6.79±1.65*** | 35.74 |
| Isovaleryl spiramycin III | 25 | 6/6 | 106.47±6.86 | 1479.90±458.97* | 18.90±3.60 | 73.13 |
| | 50 | 6/6 | 96.82±8.52 | 944.60±112.60** | 9.76±1.44** | 51.33 |
| | 100 | 6/6 | 103.26±9.71 | 635.26±78.22*** | 6.18±0.61*** | 34.20 |
| *p<0.05 compared with the model group; **p<0.01 compared with the model group, ***p<0.001 compared with the model group | | | | | | |

### 16. Inhibition of isovaleryl spiramycin I, II and III on human renal cell adenocarcinoma in nude mice model

**Establishment of a mouse solid tumor model**

769-P cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: a model group, a cyclophosphamide group, isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model

control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 34, and Table 35).

The tumor growth inhibition rates of isovaleryl spiramycin I groups with low, medium and high doses are 33.68%, 47.33% and 68.82%, respectively.

The tumor growth inhibition rates of isovaleryl spiramycin II groups with low, medium and high doses are 38.29%, 47.61% and 61.79%, respectively.

The tumor growth inhibition rates of isovaleryl spiramycin III groups with low, medium and high doses are 40.87%, 60.50% and 64.46%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are 75.78%, 57.12% and 36.88%, respectively.

The relative tumor proliferation rates of isovaleryl spiramycin II groups with low, medium and high doses are 68.22%, 42.64% and 34.76%, respectively.

The relative tumor proliferation rates of isovaleryl spiramycin III groups with low, medium and high doses are 61.59%, 51.59% and 35.55%, respectively.

There are no significant changes in the mouse weight of isovaleryl spiramycin I, II and III groups with low, medium and high doses compared with the model group.

Table 34. Effect of isovaleryl spiramycin I, II and III on the inhibition rate of transplanted tumor of human renal cell adenocarcinoma cell 769-P in nude mice ($\bar{x}\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.79±1.17 | 21.83±1.32 | 2.06±0.08*** | - |
| Cyclophos phamide | 30 | 6/6 | 21.61±1.00 | 21.58±0.81 | 0.6±0.03*** | 70.52 |
| Isovaleryl spiramycin I | 25 | 6/6 | 21.44±1.45 | 21.87±0.89 | 1.37±0.55* | 33.68 |
| | 50 | 6/6 | 21.79±1.37 | 22.00±0.94 | 1.09±0.48** | 47.33 |
| | 100 | 6/6 | 21.39±1.24 | 21.88±0.54 | 0.64±0.01*** | 68.82 |
| Isovaleryl spiramycin II | 25 | 6/6 | 22.17±1.47 | 22.05±0.71 | 1.27±0.42** | 38.29 |
| | 50 | 6/6 | 22.07±1.52 | 22.42±0.54 | 1.3±0.23*** | 47.61 |
| | 100 | 6/6 | 21.96±1.19 | 22.33±0.41 | 0.79±0.06*** | 61.79 |
| Isovaleryl spiramycin III | 25 | 6/6 | 23.09±1.90 | 22.72±0.78 | 1.22±0.46** | 40.87 |
| | 50 | 6/6 | 22.90±1.00 | 21.43±1.08 | 0.82±0.06*** | 60.50 |
| | 100 | 6/6 | 20.49±1.14 | 22.28±1.72 | 0.73±0.08*** | 64.46 |
| *p<0.05 compared with the model group, **p<0.01 compared with the model group, ***p<0.001 compared with the model group | | | | | | |

Table 35. Effect of isovaleryl spiramycin I, II and III on the volume change of transplanted tumor of human renal cell adenocarcinoma cell 769-P in nude mice ($\bar{x}\pm s$)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferat ion Rate (T/C) |
|---|---|---|---|---|---|---|
| Model Group | 0 | 6/6 | 112.53±10.69 | 2076.78±168.33 | 18.45±1.79 | -- |
| Cyclophos phamide | 30 | 6/6 | 107.92±15.94 | 704.00±45.50*** | 6.52±0.90*** | 35.34 |
| Isovaleryl spiramycin I | 25 | 6/6 | 106.47±6.86 | 1489.03±450.17* | 13.99±3.52 | 75.78 |
| | 50 | 6/6 | 96.82±8.51 | 1020.75±282.92* | 10.54±2.65* | 57.12 |
| | 100 | 6/6 | 103.26±9.71 | 702.74±37.66*** | 6.81±0.30*** | 36.88 |
| Isovaleryl spiramycin II | 25 | 6/6 | 162.50±59.24 | 2045.85±301.35 | 12.59±5.32 | 68.22 |
| | 50 | 6/6 | 193.10±35.41 | 1519.68±239.38** | 7.87±2.29*** | 42.64 |
| | 100 | 6/6 | 109.14±10.13 | 700.14±100.08*** | 6.42±0.99*** | 34.76 |
| Isovaleryl spiramycin III | 25 | 6/6 | 146.32±43.02 | 1663.32±445.50* | 11.37±1.469* | 61.59 |
| | 50 | 6/6 | 180.43±51.70 | 1717.76±524.33** | 9.52±1.67** | 51.59 |
| | 100 | 6/6 | 104.38±12.92 | 684.88±62.28*** | 6.56±0.28*** | 36.55 |
| *p<0.05 compared with the model group; **p<0.01 compared with the model group, ***p<0.001 compared with the model group | | | | | | |

**17. Inhibition of isovaleryl spiramycin I, II and III on human HT-29 in nude mice model**

**Establishment of a mouse solid tumor model**

HT-29 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the

mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 36, and Table 37).

The tumor growth inhibition rates of isovaleryl spiramycin I groups with low, medium and high doses are 16.10%, 49.72% and 70.14%, respectively.

The tumor growth inhibition rates of isovaleryl spiramycin II groups with low, medium and high doses are 16.24%, 32.41% and 55.74%, respectively.

The tumor growth inhibition rates of isovaleryl spiramycin III groups with low, medium and high doses are 19.22%, 41.35% and 63.19%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 81.60%, 49.22% and 29.11%, respectively.

The relative tumor proliferation rates of isovaleryl spiramycin II groups with low, medium and high doses are 84.18%, 79.34% and 44.05%, respectively.

The relative tumor proliferation rates of isovaleryl spiramycin III groups with low, medium and high doses are 85.54%, 53.48% and 35.63%, respectively.

There are no significant changes in the mouse weight of isovaleryl spiramycin I, II and III groups with low, medium and high doses compared with the model group.

Table 36. Effect of isovaleryl spiramycin I, II and III on the inhibition rate of transplanted tumor of HT-29 cells in nude mice (x±s)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.29±1.40 | 21.04±1.39 | 2.35±0.44 | |
| Cyclophosphamide | 30 | 6/6 | 21.42±1.35 | 18.69±1.21 | 0.50±0.25*** | 78.87 |
| Isovaleryl spiramycin I | 25 | 6/6 | 21.41±1.41 | 20.98±0.99 | 1.97±0.31 | 16.10 |
| | 50 | 6/6 | 21.31±1.53 | 20.56±1.09 | 1.18±0.34*** | 49.72 |
| | 100 | 6/6 | 20.77±1.13 | 21.19±1.26 | 0.70±0.29*** | 70.14 |
| Isovaleryl | 25 | 6/6 | 21.04±1.28 | 20.88±1.29 | 1.97±0.34 | 16.24 |
| spiramycin II | 50 | 6/6 | 21.36±1.35 | 20.9±1.29 | 1.59±0.49* | 32.41 |
| | 100 | 6/6 | 20.80±0.89 | 21.53±1.08 | 1.04±0.40*** | 55.74 |
| Isovaleryl spiramycin III | 25 | 6/6 | 21.31±1.23 | 21.19±1.52 | 1.90±0.39 | 19.22 |
| | 50 | 6/6 | 21.22±1.24 | 21.00±0.98 | 1.38±0.37** | 41.35 |
| | 100 | 6/6 | 21.34±1.78 | 21.09±1.43 | 0.87±0.34*** | 63.19 |
| *p<0.05 compared with the model group, **p<0.01 compared with the model group, ***p<0.001 compared with the model group | | | | | | |

Table 37. Effect of isovaleryl spiramycin I, II and III on the volume change of transplanted tumor of HT-29 cells in nude mice (x±s)

| Group | Dose (mg/kg ) | Number of Animals (Start/End) | Tumor Volume (mm3) (d1) | Tumor Volume (mm3) (d30) | Relative Tumor Volume (RTV) | Relative Tumor Proliferati on Rate (T/C) |
|---|---|---|---|---|---|---|
| Model Group | 0 | 6/6 | 144.17±35.58 | 1976.26±294.65 | 14.41±3.95 | |
| Cyclophos phamide | 30 | 6/6 | 176.59±36.81 | 477.12±166.46*** | 2.86±1.44*** | 19.84 |
| Isovaleryl spiramycin I | 25 | 6/6 | 162.77±38.95 | 1803.98±271.55 | 11.76±4.03 | 81.60 |
| | 50 | 6/6 | 157.41±37.31 | 1109.16±320.27** | 7.09±1.47* | 49.22 |
| | 100 | 6/6 | 169.25±35.39 | 648.93±198.53*** | 4.19±2.23** | 29.11 |
| Isovaleryl spiramycin II | 25 | 6/6 | 163.26±38.80 | 1885.85±219.49* | 12.13±3.40 | 84.18 |
| | 50 | 6/6 | 142.56±22.44 | 1568.80±603.77* | 11.43±5.13 | 79.34 |
| | 100 | 6/6 | 156.45±37.20 | 921.49±299.38*** | 6.35±2.87** | 44.05 |
| Isovaleryl spiramycin III | 25 | 6/6 | 154.49±33.07 | 1803.16±368.50* | 12.32±4.51 | 85.54 |
| | 50 | 6/6 | 161.76±30.69 | 1252.64±404.06* | 7.70±1.65* | 53.48 |
| | 100 | 6/6 | 160.38±38.86 | 826.76±259.70*** | 5.13±1.13** | 35.63 |
| *p<0.05 compared with the model group; **p<0.01 compared with the model group, ***p<0.001 compared with the model group | | | | | | |

**18. Inhibition of isovaleryl spiramycin I, II and III on human HL-60 in nude mice model**

**Establishment of a mouse solid tumor model**

HL-60 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 38, and Table 39).

The tumor growth inhibition rates of isovaleryl spiramycin I groups with low, medium and high doses are 11.57%, 34.78% and 64.19%, respectively.

The tumor growth inhibition rates of isovaleryl spiramycin II groups with low, medium and high doses are 15.92%, 29.48% and 51.81%, respectively.

The tumor growth inhibition rates of isovaleryl spiramycin III groups with low, medium and high doses are 17.10%, 29.92% and 55.05%, respectively.

The tumor volume and relative tumor volume of isovaleryl spiramycin I, II and III groups with low, medium and high doses are significantly lower than those of the model group (P<0.05).

The relative tumor proliferation rates of isovaleryl spiramycin I groups with low, medium and high doses are 84.96%, 59.54% and 32.70%, respectively.

The relative tumor proliferation rates of isovaleryl spiramycin II groups with low, medium and high doses are 75.39%, 65.18% and 41.36%, respectively.

The relative tumor proliferation rates of isovaleryl spiramycin III groups with low, medium and high doses are 76.09%, 61.90% and 39.87%, respectively.

There are no significant changes in isovaleryl spiramycin I, II and III groups with mouse weight of the low, medium and high doses compared with the model group.

Table 38. Effect of isovaleryl spiramycin I, II and III on the inhibition rate of transplanted tumor of HL60 cells in nude mice (x±s)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 20.85±1.36 | 20.71±1.39 | 2.26±0.34 | |
| Cyclophos phamide | 30 | 6/6 | 20.65±1.47 | 18.47±0.91 | 0.67±0.30*** | 70.30 |
| Isovaleryl spiramycin I | 25 | 6/6 | 20.75±1.20 | 20.66±1.08 | 2.00±0.34 | 11.57 |
| | 50 | 6/6 | 20.4±1.60 | 20.35±1.20 | 1.48±0.38** | 34.78 |
| | 100 | 6/6 | 20.63±1.33 | 20.56±1.77 | 0.81±0.42*** | 64.19 |
| Isovaleryl spiramycin II | 25 | 6/6 | 20.78±1.31 | 20.59±1.54 | 1.90±0.33 | 15.92 |
| | 50 | 6/6 | 21.01±1.20 | 20.72±1.29 | 1.60±0.45* | 29.48 |
| | 100 | 6/6 | 20.65±1.27 | 20.32±1.55 | 1.09±0.34*** | 51.81 |
| Isovaleryl spiramycin III | 25 | 6/6 | 20.60±1.57 | 20.67±1.51 | 1.88±0.25* | 17.10 |
| | 50 | 6/6 | 20.82±1.42 | 20.59±1.36 | 1.59±0.45* | 29.92 |
| | 100 | 6/6 | 20.21±1.87 | 20.25±1.22 | 1.01±0.41*** | 55.05 |
| *p<0.05 compared with the model group, **p<0.01 compared with the model group, ***p<0.001 compared with the model group | | | | | | |

Table 39. Effect of isovaleryl spiramycin I, II and III on the volume change of transplanted tumor of HL60 cells in nude mice (x±s)

| Group | Dose (mg/kg) | Number of Animals (Start/End) | Tumor Volume (mm3) (d1) | Tumor Volume (mm3) (d30) | Relative Tumor Volume (RTV) | Relativ e Tumor Prolifer ation Rate (T/C) |
|---|---|---|---|---|---|---|
| Model Group | 0 | 6/6 | 186.92±35.21 | 2009.89±276.25 | 11.25±3.65 | |
| Cyclophos phamide | 30 | 6/6 | 180.63±29.97 | 466.98±255.88*** | 2.78±1.89** | 24.69 |
| Isovaleryl spiramycin I | 25 | 6/6 | 182.56±25.80 | 1702.01±305.75 | 9.56±2.41 | 84.96 |
| | 50 | 6/6 | 189.04±24.48 | 1230.70±332.94** | 6.70±2.58* | 59.54 |
| | 100 | 6/6 | 183.82±20.41 | 657.95±411.53*** | 3.68±2.51** | 32.70 |
| Isovaleryl spiramycin II | 25 | 6/6 | 189.51±28.58 | 1596.76±293.76* | 8.48±1.37 | 75.39 |
| | 50 | 6/6 | 184.06±32.38 | 1344.04±466.62* | 7.33±2.36 | 65.18 |
| | 100 | 6/6 | 184.80±33.56 | 834.34±308.27*** | 4.65±1.77** | 41.36 |
| Isovaleryl spiramycin III | 25 | 6/6 | 184.12±22.53 | 1543.27±190.73* | 8.56±1.82 | 76.09 |
| | 50 | 6/6 | 189.31±24.59 | 1308.87±436.30* | 6.96±2.25* | 61.90 |
| | 100 | 6/6 | 188.01±27.66 | 789.65±404.78*** | 4.48±2.94** | 39.87 |
| *p<0.05 compared with the model group; **p<0.01 compared with the model group, ***p<0.001 compared with the model group | | | | | | |

**19. Inhibition of isovaleryl spiramycin I, II and III on human thyroid cancer in nude mice model**

**Establishment of a mouse solid tumor model**

TPC-1 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV×100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) × 100%.

The test results show that compared with the control group, each drug-administered group has a certain degree of inhibition on the tumor growth inhibition rate, tumor volume, relative tumor volume and relative tumor proliferation rate (see Table 40, and Table 41).

The tumor growth inhibition rates of isovaleryl spiramycin I groups with low, medium and high doses are 23.88%, 57.28% and 67.49%, respectively.

The tumor growth inhibition rates of isovaleryl spiramycin II groups with low, medium and high doses are 20.91%, 31.61% and 62.04%, respectively.

The tumor growth inhibition rates of isovaleryl spiramycin III groups with low, medium and high doses are 39.06%, 60.25% and 62.94%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 65.45%, 43.43% and 28.11%, respectively.

The relative tumor proliferation rates of isovaleryl spiramycin II groups with low, medium and high doses are 86.15%, 59.08% and 38.61%, respectively.

The relative tumor proliferation rates of isovaleryl spiramycin III groups with low, medium and high doses are 61.93%, 45.01% and 34.75%, respectively.

There are no significant changes in the mouse weight of isovaleryl spiramycin I, II and III groups with low, medium and high doses compared with the model group.

Table 40. Effect of isovaleryl spiramycin I, II and III on the inhibition rate of transplanted tumor of human thyroid cancer cell TPC-1 in nude mice (x±s)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 21.40±1.74 | 24.456±1.46 | 2.42±0.22 | -- |
| Cyclophos phamide | 30 | 6/6 | 21.56±1.54 | 19.16±1.75 | 0.69±0.37*** | 71.29 |
| Isovaleryl spiramycin I | 25 | 6/6 | 21.75±1.23 | 24.41±1.32 | 1.84±0.25** | 23.88 |
| | 50 | 6/6 | 21.53±1.43 | 25.77±1.10 | 1.03±0.44*** | 57.28 |
| | 100 | 6/6 | 21.76±1.08 | 24.12±1.20 | 0.79±0.43*** | 67.49 |
| Isovaleryl spiramycin II | 25 | 6/6 | 20.54±1.45 | 21.16±1.37 | 1.91±0.33* | 20.91 |
| | 50 | 6/6 | 20.07±1.13 | 12.45±1.63 | 1.65±0.32** | 31.61 |
| | 100 | 6/6 | 21.78±1.53 | 12.11±1.20 | 0.92±0.42*** | 62.04 |

(continued)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Isovaleryl spiramycin III | 25 | 6/6 | 21.13±1.48 | 23.49±1.02 | 1.47±0.44** | 39.06 |
| | 50 | 6/6 | 21.43±1.85 | 24.06±1.71 | 0.96±0.43*** | 60.25 |
| | 100 | 6/6 | 20.76±2.13 | 23.72±1.60 | 0.90±0.44*** | 62.94 |
| *p<0.05 compared with the model group, **p<0.01 compared with the model group, ***p<0.001 compared with the model group | | | | | | |

Table 41. Effect of isovaleryl spiramycin I, II and III on the volume change of transplanted tumor of human thyroid cancer cell TPC-1 in nude mice (x±s)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferati on Rate (T/C) |
|---|---|---|---|---|---|---|
| Model Group | 0 | 6/6 | 119.28±9.59 | 1948.78±84.91 | 16.46±1.92 | -- |
| Cyclophos | 30 | 6/6 | 117.10±4.88 | 724.64±285.14*** | 6.16±2.29*** | 37.40 |
| phamide | | | | | | |
| Isovaleryl spiramycin I | 25 | 6/6 | 115.05±9.93 | 1249.28±337.45** | 10.77±2.47** | 65.45 |
| | 50 | 6/6 | 119.45±4.30 | 847.07±356.87*** | 7.15±3.19*** | 43.43 |
| | 100 | 6/6 | 112.69±11.49 | 490.15±322.14*** | 4.63±3.34*** | 28.11 |
| Isovaleryl spiramycin II | 25 | 6/6 | 119.12±3.42 | 1689.63±210.37* | 14.18±1.70 | 86.15 |
| | 50 | 6/6 | 118.54±6.49 | 1148.57±344.78** | 9.72±3.02** | 59.08 |
| | 100 | 6/6 | 117.30±6.48 | 754.42±320.69*** | 6.36±2.38*** | 38.61 |
| Isovaleryl spiramycin III | 25 | 6/6 | 119.35±3.84 | 1210.76±295.50** | 10.19±2.79** | 61.93 |
| | 50 | 6/6 | 116.75±5.22 | 866.73±306.25*** | 7.41±2.51*** | 45.01 |
| | 100 | 6/6 | 115.43±7.58 | 654.78±338.68*** | 5.72±3.04*** | 34.75 |
| *p<0.05 compared with the model group; **p<0.01 compared with the model group, ***p<0.001 compared with the model group | | | | | | |

**20. Inhibition of isovaleryl spiramycin I, II and III on human bladder cancer in nude mice model**

**Establishment of a mouse solid tumor model**

T-24 cells in a logarithmic growth phase were taken and subjected to a trypan blue exclusion experiment showing that the cell viability was more than 95%, then the cells were subjected to trypsinization, centrifugation, and supernatant removal. Then cell concentration was adjusted to $1\times10^7$/ml with matrigel, then each nude mouse was inoculated subcutaneously with 0.2ml of cells at its right armpit and recorded as the first day of inoculation. When the tumor grows to be greater than or equal to 100 $mm^3$, the mice were randomly divided into 11 groups with 6 mice in each group: isovaleryl spiramycin I groups with doses of 25, 50 and 100 mg/kg, isovaleryl spiramycin II groups with doses of 25, 50 and 100 mg/kg, and isovaleryl spiramycin III groups with doses of 25, 50 and 100 mg/kg. Each group was continuously administered intragastrically for 30 days with a dose of 20 ml/kg. The mice were sacrificed the next day after drug withdrawal and the indicators were tested. The long diameter and short diameter of the tumor, and the body weight of each mouse were recorded every 3 days from drug administration to nude mouse sacrifice.

**Calculation of tumor volume and relative tumor proliferation rate**

The body weight of the nude mice and the long diameter (a) and short diameter (b) of the transplanted tumor were measured every 3 days, and the tumor volume (v), relative tumor volume (RTV) and relative tumor proliferation rate (T/C) are calculated respectively according to the following formulas, wherein $V=a\times b^2/2$; RTV = $V/V_0$ ($V_0$ is the tumor volume before administration, V is the tumor volume before sacrifice), and T/C (%) = treatment group RTV/model control group RTV$\times$100%.

**Calculation of tumor growth inhibition rate**

Each mouse was weighed and sacrificed. After the tumor was completely stripped off from the body of the mouse, the non-tumor tissues such as blood stains and fat were removed to weigh the tumor and calculate the tumor growth inhibition rate. The average tumor weight of each group of mice is used as an indicator of efficacy. Tumor growth inhibition rate (%) = (1-the average tumor weight of the treatment group/ the average tumor weight of the model group) $\times$ 100%.

The test results show that compared with the control group, each drug-administered group has a certain degree of inhibition on the tumor growth inhibition rate, tumor volume, relative tumor volume and relative tumor proliferation rate (see Table 42, and Table 43).

The tumor growth inhibition rates of isovaleryl spiramycin I groups with low, medium and high doses are 20.55%, 49.20% and 65.84%, respectively.

The tumor growth inhibition rates of isovaleryl spiramycin II groups with low, medium and high doses are 15.57%, 41.99% and 59.25%, respectively.

The tumor growth inhibition rates of isovaleryl spiramycin III groups with low, medium and high doses are 21.00%, 44.84% and 61.30%, respectively.

The tumor volume and relative tumor volume of isovaleryl spiramycin I, II and III groups with low, medium and high doses are significantly lower than those of the model group ($P<0.05$).

The relative tumor proliferation rates of isovaleryl spiramycin I groups with low, medium and high doses are 77.78%, 58.92% and 33.95%, respectively.

The relative tumor proliferation rates of isovaleryl spiramycin II groups with low, medium and high doses are 81.39%, 64.89% and 46.70%, respectively.

The relative tumor proliferation rates of isovaleryl spiramycin III groups with low, medium and high doses are 80.34%, 62.35% and 39.39%, respectively.

There are no significant changes in the mouse weight of isovaleryl spiramycin I, II and III groups with low, medium and high doses compared with the model group.

Table 42. Effect of isovaleryl spiramycin I, II and III on the inhibition rate of transplanted tumor of human bladder cancer cell T-24 in nude mice ($x\pm s$)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Model Group | 0 | 6/6 | 19.69±0.72 | 24.75±2.70 | 1.87±0.21 | |

(continued)

| Group | Dose (mg/kg) | Number of Animals (Start/End) | Body Weight (g) (d1) | Body Weight (g) (d30) | Tumor Weight (g) | Inhibition Rate (%) |
|---|---|---|---|---|---|---|
| Cyclophos phamide | 30 | 6/6 | 19.53±0.61 | 27.28±1.43 | 0.51±0.12*** | 73.04 |
| Isovaleryl spiramycin I | 25 | 6/6 | 19.51±0.58 | 23.89±1.18 | 1.49±0.23* | 20.55 |
| | 50 | 6/6 | 19.60±0.70 | 25.41±0.92 | 0.95±0.38*** | 49.20 |
| | 100 | 6/6 | 19.49±0.54 | 24.6±2.08 | 0.64±0.18*** | 65.84 |
| Isovaleryl spiramycin II | 25 | 6/6 | 19.91±0.28 | 24.81±2.42 | 1.58±0.28 | 15.57 |
| | 50 | 6/6 | 19.74±0.82 | 25.84±0.73 | 1.09±0.42** | 41.99 |
| | 100 | 6/6 | 19.51±0.50 | 25.92±1.35 | 0.76±0.19*** | 59.25 |
| Isovaleryl spiramycin III | 25 | 6/6 | 19.57±0.52 | 25.72±1.53 | 1.48±0.43* | 21.00 |
| | 50 | 6/6 | 19.61±0.44 | 26.47±0.52 | 1.03±0.42** | 44.84 |
| | 100 | 6/6 | 19.90±0.58 | 25.05±0.93 | 0.73±0.20*** | 61.30 |
| *p<0.05 compared with the model group, **p<0.01 compared with the model group, ***p<0.001 compared with the model group | | | | | | |

Table 43. Effect of isovaleryl spiramycin I, II and III on the volume change of transplanted tumor of human bladder cancer cell T-24 in nude mice (x±s)

| Group | Dose (mg/kg) | Number of Animals (Start/End) | Tumor Volume (mm3) (d1) | Tumor Volume (mm3) (d30) | Relative Tumor Volume (RTV) | Relative Tumor Proliferati on Rate (T/C) |
|---|---|---|---|---|---|---|
| Model Group | 0 | 6/6 | 134.41±15.31 | 1772.51±198.69 | 13.21±0.72 | |
| Cyclophos phamide | 30 | 6/6 | 136.87±19.82 | 389.34±82.07 | 2.85±0.50 | 21.60 |
| Isovaleryl spiramycin I | 25 | 6/6 | 139.84±15.80 | 1428.71±156.12 | 10.28±1.12 | 77.78 |
| | 50 | 6/6 | 131.81±7.15 | 1025.16±174.78 | 7.78±1.27 | 58.92 |
| | 100 | 6/6 | 134.21±14.43 | 606.16±136.06 | 4.48±0.75 | 33.95 |
| Isovaleryl spiramycin II | 25 | 6/6 | 132.64±19.75 | 1414.36±188.91 | 10.75±1.34 | 81.39 |
| | 50 | 6/6 | 133.67±5.07 | 1142.42±146.91 | 8.57±1.31 | 64.89 |
| | 100 | 6/6 | 133.57±18.08 | 809.94±131.43 | 6.17±1.26 | 46.70 |
| Isovaleryl spiramycin III | 25 | 6/6 | 135.55±18.08 | 1427.95±311.97 | 10.61±2.24 | 80.34 |
| | 50 | 6/6 | 136.32±15.54 | 1123.28±140.84 | 8.24±0.38 | 62.35 |
| | 100 | 6/6 | 134.53±20.46 | 697.48±130.89 | 5.20±0.71 | 39.39 |
| *p<0.05 compared with the model group; **p<0.01 compared with the model group, ***p<0.001 compared with the model group | | | | | | |

**21. Inhibition of transplanted tumors of mouse $H_{22}$ liver cancer and mouse Lewis lung cancer**

**Establishment of a mouse solid tumor model:**

The $H_{22}$ cell strains cryopreserved in liquid nitrogen were resuscitated in Kunming mice. After 3 generations, the ascites of Kunming mice were taken and placed in a 50 ml centrifuge tube in which 10 ml of 0.9% normal saline was added, and then centrifuging was performed at 1000rpm for 5 min at room temperature, and the obtained supernatant was removed. Then 10 ml of 0.9% normal saline was added to the tube to blow and mix well, and then the mixture was diluted to $5\times10^6$ live cells /ml with normal saline after counting. The tube was stored in ice water, and 75% ethanol was used to disinfect the skin under the right armpits of the mice. Each Kunming mouse was soon inoculated subcutaneously with 0.2ml of the cells at the armpit of the right forelimb.

Lewis lung cancer cells were cultured in a RPMI 1640 culture medium containing 10% fetal bovine serum at 37°C in a 5% $CO_2$ incubator. The cells in the logarithmic growth phase were subjected to trypsinization with 0.25% trypsin, then the cells were collected to be centrifugated to remove the obtained supernatant, then were washed twice with sterile normal saline. And then the cells were suspended in the normal saline to be subjected to a trypan blue staining assay which shows that the cell viability was greater than 95%, and then cell counting was performed. The Lewis cells were adjusted to $1\times10^7$/mL in concentration and stored in ice water. 75% ethanol was used to disinfect the skin under the right armpits of the mice, and each C57BL/6 mouse was soon inoculated subcutaneously with 0.2 ml of the cells at its right armpit.

**Mouse grouping and administration method**

In the $H_{22}$ liver cancer model, the mice inoculated with the tumor were randomly divided into groups with 10 mice in each group on the next day of inoculation. The groups included: a model control group, a positive drug cyclophosphamide control group (CTX, 26 mg/kg), isovaleryl spiramycin I groups with doses of 13, 26 and 52mg/kg; isovaleryl spiramycin II groups with doses of 13, 26 and 52mg/kg; isovaleryl spiramycin III groups with doses of 13, 26 and 52mg/kg. Each group was continuously administered intragastrically for 7 days with a dose of 20 ml/kg.

In the Lewis lung cancer model, the mice inoculated with the tumor were randomly divided into groups with 10 mice in each group the next day of inoculation. The groups included: a model control group, a positive drug cyclophosphamide control group (CTX, 30 mg/kg), isovaleryl spiramycin I groups with doses of 13, 26 and 52mg/kg; isovaleryl spiramycin II groups with doses of 13, 26 and 52mg/kg; isovaleryl spiramycin III groups with doses of 13, 26 and 52mg/kg. Each group was continuously administered intragastrically for 15 days with a dose of 20 ml/kg.

**Calculation of the tumor inhibition rate:**

The tumor-bearing mice were weighed and sacrificed the next day after the last administration. The subcutaneous tumors were dissected and weighed. The average tumor weight of each group is calculated, and the tumor inhibition rate is calculated.

$$\text{Tumor inhibition rate} = (1\text{-}T/C) \times 100\%$$

T: average tumor weight of the drug-administered group; C: average tumor weight of the blank control group.

Results:

**1. Inhibition of isovaleryl spiramycin I, II and III on the transplanted tumor of mouse $H_{22}$ liver cancer**

As can be seen from the results of Table 44, the tumor inhibition rate of the positive drug cyclophosphamide control group to Kunming mouse $H_{22}$ liver cancer is 73.03%. Isovaleryl spiramycin I groups with doses of 13, 26 and 52mg/kg, isovaleryl spiramycin II groups with doses of 13, 26 and 52mg/kg, and isovaleryl spiramycin III groups with doses of 13, 26 and 52mg/kg significantly inhibit the growth of $H_{22}$ liver cancer in mice.

The positive drug cyclophosphamide group shows a slight decrease in weight compared with the normal control group. The weight of the mice in each of the isovaleryl spiramycin I, II and III groups increases compared with that before the administration, but there is no significant difference compared with the model control group.

Table 44. Inhibition of isovaleryl spiramycin I, II and III on the transplanted tumor of mouse $H_{22}$ liver cancer (x±s)

| Group | Dose (mg/kg) | Number of Animals (Start/End) | Body Weight (g) (d1) | Body Weight (g) (d7) | Tumor (g) | Inhibition Rate (%) |
|---|---|---|---|---|---|---|
| Model Group | 0 | 10/10 | 23.83 ±0.43 | 26.13 ±1.26 | 1.52±0.58 | |
| Cyclophos phamide | 26 | 10/10 | 23.72 ±1.79 | 23.68 ±1.32 | 0.41±0.27*** | 73.03 |
| Isovaleryl spiramycin I | 13 | 10/10 | 24.55 ±1.00 | 26.23 ±0.79 | 0.88±0.38* | 42.11 |
| | 26 | 10/10 | 23.83 ±2.36 | 26.68 ±1.85 | 0.68±0.24** | 55.26 |
| | 52 | 10/10 | 24.02 ±2.83 | 27.87 ±1.57 | 0.52±0.68*** | 65.79 |
| Isovaleryl spiramycin II | 13 | 10/10 | 24.22 ±2.15 | 27.27 ±2.20 | 1.22±0.31 | 19.74 |
| | 26 | 10/10 | 24.72 ±1.69 | 27.35 ±0.80 | 1.06±0.33* | 30.26 |
| | 52 | 10/10 | 23.45 ±1.69 | 27.02 ±0.90 | 0.83±0.46** | 45.39 |
| Isovaleryl spiramycin III | 13 | 10/10 | 23.67 ±3.73 | 26.33 ±1.43 | 1.01±0.22* | 33.55 |
| | 26 | 10/10 | 24.13 ±1.46 | 27.47 ±1.21 | 0.85±0.34* | 44.08 |
| | 52 | 10/10 | 24.32 ±1.12 | 27.35 ±0.80 | 0.73±0.45** | 51.97 |
| *p<0.05 compared with the model group; **p<0.01 compared with the model group; ## p<0.05 compared with the cyclophosphamide group | | | | | | |

Table 45. Effect of isovaleryl spiramycin I, II and III on the tumor volume of transplanted tumor of KM mouse $H_{22}$ liver cancer (x±s)

| Group | Dose (mg/kg) | Number of Animals (Start/E nd) | Tumor Volume (mm3) (d1) | Tumor Volume (mm3) (d30) | Relative Tumor Volume (RTV) | Relative Tumor Prolifer ation Rate (T/C) |
|---|---|---|---|---|---|---|
| Model Group | 0 | 6/6 | 118.93 ±13.02 | 1183.27 ±297.43 | 9.93 ±2.45 | |
| Cyclophos phamide | 30 | 6/6 | 119.60±31.11 | 307.34±79.91*** | 2.86±1.44 *** | 28.78 |
| Isovaleryl spiramycin I | 12.5 | 6/6 | 120.26 ±24.42 | 657.57 ±231.56** | 5.41 ±1.42 ** | 54.47 |
| | 25 | 6/6 | 118.32 ±11.90 | 563.69 ±63.08** | 4.79 ±0.64 ** | 48.27 |
| | 50 | 6/6 | 120.68 ±13.76 | 359.74 ±80.81*** | 2.96 ±0.39*** | 29.78 |
| Isovaleryl spiramycin II | 12.5 | 6/6 | 120.80 ±18.48 | 931.38 ±141.51* | 7.83 ±1.50 * | 78.85 |
| | 25 | 6/6 | 118.02 ±10.35 | 779.30 ±292.34** | 6.56 ±2.19 ** | 66.04 |
| | 50 | 6/6 | 118.18 ±14.68 | 659.02 ±138.35** | 5.58 ±0.95 ** | 56.20 |

(continued)

| Group | Dose (mg/kg) | Number of Animals (Start/E nd) | Tumor Volume (mm3) (d1) | Tumor Volume (mm3) (d30) | Relative Tumor Volume (RTV) | Relative Tumor Prolifer ation Rate (T/C) |
|---|---|---|---|---|---|---|
| Isovaleryl spiramycin | 12.5 | 6/6 | 121.58 ±13.44 | 824.36 ±143.90* | 6.92 ±1.88* | 69.68 |
| | 25 | 6/6 | 118.16 ±21.72 | 650.32 ±171.23** | 5.65 ±1.74 ** | 56.90 |
| III | 50 | 6/6 | 122.15 ±49.44 | 468.19 ±110.04*** | 4.31 ±1.65 *** | 43.45 |
| *p<0.05 compared with the model group; **p<0.01 compared with the model group, ***p<0.001 compared with the model group | | | | | | |

**2. Inhibition of isovaleryl spiramycin I, II and III on the transplanted tumor of mouse Lewis lung cancer**

As can be seen from the results of Table 46, the tumor inhibition rate of the positive drug cyclophosphamide control group to mouse Lewis lung cancer is 76.43%. Isovaleryl spiramycin I groups with doses of 13, 26 and 52mg/kg, isovaleryl spiramycin II groups with doses of 13, 26 and 52mg/kg, and isovaleryl spiramycin III groups with doses of 13, 26 and 52mg/kg significantly inhibit the growth of Lewis lung cancer in mice. The weight of the mice in each of the isovaleryl spiramycin I, II and III groups increases compared with that before the administration, but there is no significant difference compared with the model control group.

Table 46. Inhibition of isovaleryl spiramycin I, II and III on the transplanted tumor of mouse Lewis lung cancer (x±s)

| Group | Dose (mg/kg) | Number of Animals (Start/End) | Body Weight (g) (d1) | Body Weight (g) (d15) | Tumor Weight (g) | Inhibition Rate (%) |
|---|---|---|---|---|---|---|
| Model Group | 0 | 10/10 | 19.60 ±1.07 | 22.70 ±1.28 | 1.60 ±0.56 | |
| Cyclophosph amide | 30 | 10/10 | 19.62 ±1.01 | 21.38 ±0.67 | 0.38 ±0.07*** | 76.43 |
| Isovaleryl spiramycin I | 13 | 10/10 | 19.93 ±1.20 | 22.70 ±0.87 | 0.85 ±0.24* | 46.94 |
| | 26 | 10/10 | 19.45 ±0.68 | 23.43 ±1.41 | 0.59 ±0.06** | 62.97 |
| | 52 | 10/10 | 19.87 ±1.17 | 23.25 ±0.43 | 0.45 ±0.14*** | 71.83 |
| Isovaleryl spiramycin II | 13 | 10/10 | 20.33 ±0.64 | 22.17 ±1.74 | 0.98 ±0.37 | 38.67 |
| | 26 | 10/10 | 19.62 ±1.43 | 22.38 ±0.96 | 0.89 ±0.18 * | 44.26 |
| | 52 | 10/10 | 20.05 ±0.67 | 22.75 ±0.95 | 0.70 ±0.21** | 56.34 |

(continued)

| Group | Dose (mg/kg) | Number of Animals (Start/End) | Body Weight (g) (d1) | Body Weight (g) (d15) | Tumor Weight (g) | Inhibition Rate (%) |
|---|---|---|---|---|---|---|
| Isovaleryl spiramycin III | 13 | 10/10 | 19.62 ±1.30 | 22.52 ±0.57 | 1.03 ±0.18 * | 35.46 |
| | 26 | 10/10 | 19.58 ±0.63 | 22.28 ±0.66 | 0.82 ±0.34 * | 48.96 |
| | 52 | 10/10 | 20.00 ±0.60 | 22.42 ±1.66 | 0.68 ±0.18** | 57.54 |
| *p<0.05 compared with the model group; **p<0.01 compared with the model group; ##p<0.05 compared with the cyclophosphamide group | | | | | | |

Table 47. Effect of isovaleryl spiramycin I, II and III on the tumor volume of transplanted tumor of KM mouse $H_{22}$ cells (x±s)

| Group | Dose (mg/kg) | Numb er of Anim als (Start/ End) | Tumor Volume (mm3) (d1) | Tumor Volume (mm3) (d30) | Relative Tumor Volume (RTV) | Relative Tumor Proliferati on Rate (T/C) |
|---|---|---|---|---|---|---|
| Model Group | 0 | 6/6 | 104.31 ±16.13 | 1818.28 ±397.72 | 16.01 ±4.15 | |
| Cycloph osphami de | 30 | 6/6 | 104.35 ±30.89 | 371.39 ±112.68*** | 3.93 ±1.86*** | 24.55 |
| Isovalery 1 spiramyc in I | 12.5 | 6/6 | 104.68 ±20.89 | 955.73 ±330.24** | 9.22 ±2.95** | 57.56 |
| | 25 | 6/6 | 104.31 ±19.76 | 756.20 ±145.84** | 7.29 ±0.82** | 45.51 |
| | 50 | 6/6 | 103.79 ±19.55 | 465.26 ±52.94*** | 4.65 ±1.27*** | 29.05 |
| Isovalery 1 spiramyc in II | 12.5 | 6/6 | 104.18 ±19.49 | 1304.48 ±379.51* | 12.89 ±4.69* | 80.51 |
| | 25 | 6/6 | 103.63 ±19.46 | 1119.26 ±338.67* | 11.04 ±4.07* | 68.97 |
| | 50 | 6/6 | 103.38 ±18.03 | 798.42 ±189.19** | 7.81 ±1.76** | 48.76 |
| Isovalery 1 spiramyc in III | 12.5 | 6/6 | 105.02 ±31.31 | 1270.87 ±198.05* | 13.54 ±6.73* | 84.57 |
| | 25 | 6/6 | 103.29 ±29.09 | 841.92 ±385.16** | 9.35 ±5.89** | 58.38 |
| | 50 | 6/6 | 104.43 ±23.09 | 677.35 ±237.77** | 6.69 ±2.56** | 41.81 |
| *p<0.05 compared with the model group; **p<0.01 compared with the model group, ***p<0.001 compared with the model group | | | | | | |

### 22. Effect of isovaleryl spiramycin I, II and III on immune function of tumor-bearing mice

**Method**

**1. Effect on thymus index and spleen index of tumor-bearing mice**

After the tumor-bearing mice are sacrificed, the spleen and thymus are weighed, and the spleen index and thymus index are calculated.

**2. Effects on lymphocyte proliferation activity and natural killer (NK) cell activity of tumor-bearing mice**

**2.1 Preparation of spleen lymphocytes**

The serum-free RPMI 1640 medium was placed in a dish, and then the dish was placed on ice. The spleen was aseptically taken and gently ground with a sterile glass slide to prepare a single cell suspension. The single cell suspension was filtered with a double-layer sterile gauze, washed twice with serum-free RPMI1640 medium, and centrifuged at 1,500 rpm for 5 min to remove the obtained supernatant. 2 mL of red blood cell lysate was added to the treated suspension, the mixture was allowed to stand for 2 min, and then 8 mL of RPMI 1640 medium was added, centrifuging was performed at 1,500 rpm for 5 min to remove the obtained supernatant, and then washing was performed twice with the RPMI 1640 medium. Trypan blue staining was performed to count the number of live cells, and the cell viability was more than 95%. A single cell suspension was prepared by using a RPMI 1640 medium containing 10% fetal bovine serum.

**2.2 Spleen lymphocyte proliferation activity assay**

The spleen cell suspension was taken, and the cell density was adjusted to $1 \times 10^7$/mL. Each mouse was set with: A. a control well: 100 μL of RPMI 1640 medium was added; B. a concanavalin A (ConA) stimulation well: 100 μL (10 mg/L) of concanavalin A (ConA) solution was added; and C. a bacterial endotoxin (LPS) stimulating well: 100 μL (20 mg/L) of bacterial endotoxin (LPS) solution was added. The above cells were added to a 96-well plate, and then 100 μL of spleen cell suspension was added to each of the above wells. After the culture plate was transferred to a saturated humidity condition with a volume fraction of 5% $CO_2$ at 37°C for incubation for 72 h, 10 μL of MTT solution (5 g/L) was added to each well, and incubation was continued to be performed for 4 hours under the same conditions, then the culture was terminated. 100μl of a triple solution (SDS 10g, 10M HCl 0.1mL, wasobutanol 5mL, diluted with dwastilled water to 100mL) was added, and the plate was shaken for 10min to fully dissolve the crystals. The Optical Density (OD) of each well was measured at 570nm, and the lymphocyte proliferation rate was calculated. Lymphocyte proliferation rate (%) = [(T-C)/C] $\times$ 100%, wherein T was the Optical Density of the stimulation well, and C is the Optical Density of the control well.

**2.3 Natural killer (NK) cell activity assay**

The spleen cell suspension was taken, and the cell density was adjusted to $1 \times 10^7$/mL (effector cells). A suspension of K562 cells was prepared with a cell density of $1 \times 10^5$/mL (target cells). Each mouse was set with: A. effector cells: target cell well (quantity ratio 20:1) to which 20 μL of spleen cell suspension and 100 μL of K562 cell suspension were added; B. an effector cell control well to which 100 μL of spleen cell suspension and 100 μL of RPMI 1640 medium were added; and C. a target cell control well to which 100 μL of K562 cell suspension and 100 μL of RPMI 1640 medium were added. The above cells were added to a 96-well plate. After the 96-well plate was transferred to a saturated humidity condition with a volume fraction of 5% $CO_2$ at 37°C for incubation for 22 h, 10 μL of MTT solution (5 g/L) was added to each well, and incubation was continued to be performed for 4 hours under the same conditions, , then the culture was terminated. 100μl of a triple solution (SDS 10g, 10M HCl 0.1mL, isobutanol 5mL, diluted with distilled water to 100mL) was added, and the plate was shaken for 10min to fully dissolve the crystals, and the Optical Density (OD) of each well at 490 nm was measured, and the NK cell activity was calculated. NK cell activity (%) = [TO-(S-E)] /TO $\times$100%, wherein TO is the Optical Density of the target cell control well, S is the Optical Density of the effector cell control well, and E is the Optical Density of the effector cell.

Results:

**1. Effect on thymus index and spleen index of $H_{22}$ liver cancer tumor-bearing mice**

As can be seen from the results of Table 48, the thymus index and spleen index of the positive drug cyclophosphamide control group are significantly lower than those of the control group (P<0.01). The thymus indexes of the mice in the isovaleryl spiramycin I groups with doses of 13, 26 and 52mg/kg, isovaleryl spiramycin II groups with doses of 13, 26 and 52mg/kg, and isovaleryl spiramycin III groups with doses of 13, 26 and 52mg/kg have no significant change compared with that of the control group.

Table 48. Effect of isovaleryl spiramycin I, II and III on thymus index and spleen index (w) of $H_{22}$ liver tumor-bearing mice (x±s)

| Group | Dose (mg/kg) | Spleen Index (%) | Thymus Index (%) |
|---|---|---|---|
| Control Group | 0 | 0.66±0.12 | 0.13±0.05 |
| Cyclophosphamide | 26 | 0.38±0.09** | 0.04±0.02** |
| Isovaleryl spiramycin I | 13 | 0.72±0.07 | 0.15±0.07 |
| | 26 | 0.60±0.15 | 0.14±0.01 |
| | 52 | 0.86±0.25* | 0.12±0.04 |
| Isovaleryl spiramycin II | 13 | 0.59±0.24 | 0.12±0.05 |
| | 26 | 0.68±0.24 | 0.16±0.03 |
| | 52 | 0.64±0.17 | 0.17±0.10 |
| Isovaleryl spiramycin III | 13 | 0.68±0.15 | 0.15±0.06 |
| | 26 | 0.66±0.21 | 0.15±0.05 |
| | 52 | 0.65±0.26 | 0.13±0.02 |
| *p<0.05 compared with the control group; **p<0.01 compared with the control group | | | |

**2. Effect on thymus index and spleen index of Lewis lung cancer tumor-bearing mice**

As can be seen from the results in Table 49, the spleen index of the positive drug cyclophosphamide control group is significantly lower than that of the control group (P<0.01). The spleen index and thymus index of the mice in the isovaleryl spiramycin I groups with doses of 13, 26 and 52mg/kg, isovaleryl spiramycin II groups with doses of 13, 26 and 52mg/kg, and isovaleryl spiramycin III groups with doses of 13, 26 and 52mg/kg are not significantly different from those in the control group.

Table 49. Effect of isovaleryl spiramycin I, II and III on thymus index and spleen index (w) in Lewis lung cancer-bearing mice (x±s, n=6)

| Group | Dose (mg/kg) | Spleen Index (%) | Thymus Index (%) |
|---|---|---|---|
| Control Group | 0 | 0.76±0.12 | 0.12±0.05 |
| Cyclophosphamide | 26 | 0.48±0.09** | 0.06±0.02** |
| Isovaleryl spiramycin I | 13 | 0.72±0.21 | 0.12±0.03 |
| | 26 | 0.62±0.15 | 0.13±0.04 |
| | 52 | 0.77±0.14* | 0.12±0.06 |
| Isovaleryl spiramycin II | 13 | 0.75±0.24 | 0.12±0.08 |
| | 26 | 0.68±0.21 | 0.14±0.02 |
| | 52 | 0.66±0.17 | 0.15±0.09 |
| Isovaleryl spiramycin III | 13 | 0.62±0.25 | 0.12±0.10 |
| | 26 | 0.68±0.11 | 0.11±0.07 |
| | 52 | 0.74±0.23 | 0.13±0.06 |
| **p<0.01 compared with the control group; *p<0.05 compared with the control group; | | | |

### 3. Effect on NK cell activity of Lewis lung cancer tumor-bearing mice

As can be seen from the results in Table 50, the NK cell activity of the positive drug cyclophosphamide control group is significantly lower than that of the control group (P<0.05). The NK cell activities in isovaleryl spiramycin I groups with doses of 13, 26 and 52mg/kg, isovaleryl spiramycin II groups with doses of 13, 26 and 52mg/kg, and isovaleryl spiramycin III groups with doses of 13, 26 and 52mg/kg significantly increase compared with that of the control group (P<0.01).

Table 50. Effect of isovaleryl spiramycin I, II and III on the NK cell activity of Lewis lung cancer tumor-bearing (x±s, n=6)

| Group | Dose(mg/kg) | NK Cell Activity (%) |
|---|---|---|
| Control Group | 0 | 46.2±5.2 |
| Cyclophosphamide | 26 | 35.4±6.6* |
| Isovaleryl spiramycin I | 13 | 52.5±9.2 |
| | 26 | 67.0±12.1** |
| | 52 | 39.8±6.8 |
| Isovaleryl spiramycin II | 13 | 47.2±9.2 |
| | 26 | 47.0±5.5 |
| | 52 | 43.1±7.3 |
| Isovaleryl spiramycin III | 13 | 48.9±6.6 |
| | 26 | 46.0±11.8 |
| | 52 | 49.8±6.2 |
| **p<0.01 compared with the control group; *p<0.05 compared with the control group; | | |

### 4. Effect on lymphocyte proliferation activity of Lewis lung cancer tumor-bearing mice

As can be seen from the results in Table 51, the lymphocyte activity of the positive drug cyclophosphamide control group is significantly inhibited (P < 0.05). The lymphocyte activities of isovaleryl spiramycin I groups with doses of 13, 26 and 52mg/kg, isovaleryl spiramycin II groups with doses of 13, 26 and 52mg/kg, and isovaleryl spiramycin III groups with doses of 13, 26 and 52mg/kg significantly increase compared with that of the control group (P<0.05, P<0.01).

Table 51. Effect of isovaleryl spiramycin I, II and III on lymphocyte proliferation in transplanted tumor of Lewis lung cancer mice (x±s, n=6)

| Group | Dose (mg/kg) | B Lymphocyte Proliferation Activity (%) | T Lymphocyte Proliferation Activity (%) |
|---|---|---|---|
| Control Group | 0 | 30.37±10.16 | 17.60±7.39 |
| Cyclophosphamide | 26 | 11.63±4.68* | 13.24±3.72* |
| Isovaleryl spiramycin | 13 | 41.63±7.06* | 25.27±8.20** |
| I | 26 | 44.81±4.41* | 36.24±2.15** |
| | 52 | 32.71±1.84 | 22.26±4.33 |
| Isovaleryl spiramycin II | 13 | 39.88±3.57 | 16.87±3.28 |
| | 26 | 32.68±2.68 | 15.82±1.68 |
| | 52 | 35.94±3.80 | 19.28±2.35 |
| Isovaleryl spiramycin III | 13 | 33.91±2.65 | 20.17±5.18 |
| | 26 | 34.69±0.34 | 18.51±2.60 |
| | 52 | 32.28±1.27 | 18.25±1.89 |
| *p<0.05 compared with the control group; **p<0.01 compared with the control group; | | | |

**5. Effect on spleen index of A549 lung cancer tumor-bearing mice**

As can be seen from the results in Table 52, the spleen index of the positive drug cyclophosphamide control group is significantly lower than that of the control group (P<0.01). The spleen index of the mice in the isovaleryl spiramycin I groups with doses of 13, 26 and 52mg/kg, isovaleryl spiramycin II groups with doses of 13, 26 and 52mg/kg, and isovaleryl spiramycin III groups with doses of 13, 26 and 52mg/kg do not change significantly compared with that of the control group.

Table 52. Effect of isovaleryl spiramycin I, II and III on spleen index of A549 lung cancer tumor-bearing mice (x±s, n=6)

| Group | Dose (mg/kg) | Spleen Index (%) |
|---|---|---|
| Control Group | 0 | 0.31±0.04 |
| Cyclophosphamide | 26 | 0.21±0.07* |
| Isovaleryl spiramycin I | 13 | 0.32±0.12 |
| | 26 | 0.38±0.09 |
| | 52 | 0.31±0.08 |
| Isovaleryl spiramycin II | 13 | 0.35±0.06 |
| | 26 | 0.32±0.09 |
| | 52 | 0.37±0.06 |
| Isovaleryl spiramycin III | 13 | 0.32±0.02 |
| | 26 | 0.33±0.11 |
| | 52 | 0.35±0.09 |
| *p<0.05 compared with the control group | | |

The above are only preferred embodiments of the present disclosure, and are not intended to limit the present disclosure in any form. Although the present disclosure has been disclosed in the above preferred embodiments, they are not intended to limit the present disclosure.

## Claims

1. Isovaleryl spiramycin I or III for use in treating and/or preventing tumors, wherein the tumor is breast cancer, liver cancer, lung cancer, renal cancer, brain tumor, cervical cancer, prostate cancer, lymphoma, pancreatic cancer, esophageal cancer, gastric cancer, colon cancer, thyroid cancer, bladder cancer, malignant skin tumor, or lymphoma or leukemia.

2. Isovaleryl spiramycin I or III for use according to Claim 1, wherein the brain tumor is glioma or meningioma, and the gastric cancer is gastric adenocarcinoma.

3. Isovaleryl spiramycin I or III for use according to Claim 1 or 2, wherein a medicament is in various formulations including isovaleryl spiramycin I or III and pharmaceutically acceptable adjuvants, or
the medicament is in various formulations including pharmaceutically acceptable salts of isovaleryl spiramycin I or III and pharmaceutically acceptable adjuvants.

4. Isovaleryl spiramycin I or III for use according to Claim 1 or 2, wherein a medicament is in various formulations including isovaleryl spiramycin I or III, anti-tumor drugs and the pharmaceutically acceptable adjuvants, or
the medicament is in various formulations including the pharmaceutically acceptable salts of isovaleryl spiramycin I or III, anti-tumor drugs and the pharmaceutically acceptable adjuvants.

5. Isovaleryl spiramycin I or III for use according to Claim 1 or 2, wherein a medicament is a combination of a first agent and a second agent, the first agent contains isovaleryl spiramycin I or III and the pharmaceutically acceptable salts of isovaleryl spiramycin I or III, and the second agent contains an anti-tumor drug.

6. Isovaleryl spiramycin I or III for use according to Claim 4 or 5, wherein the anti-tumor drug is one or more than one drug selected from a group containing a chemotherapy drug, a radiotherapy drug, a targeted therapy drug, and an immunotherapeutic drug.

7. Isovaleryl spiramycin I or III for use according to Claim 4 or 5, wherein a dose of isovaleryl spiramycin I or III in the medicament is in a range from 5 to 1,500 mg.

8. Isovaleryl spiramycin I or III for use according to Claim 7, wherein a dose of isovaleryl spiramycin I or III in the medicament is in a range from 50 to 1,000 mg.

9. Isovaleryl spiramycin I or III for use according to Claim 8, wherein a dose of isovaleryl spiramycin I or III in the medicament is in a range from 100 to 400 mg.

## Patentansprüche

1. Isovalerylspiramycin I oder III zur Verwendung bei der Behandlung und/oder Vorbeugung von Tumoren, wobei der Tumor Brustkrebs, Leberkrebs, Lungenkrebs, Nierenkrebs, Hirntumor, Gebärmutterhalskrebs, Prostatakrebs, Lymphom, Bauchspeicheldrüsenkrebs, Speiseröhrenkrebs, Magenkrebs, Darmkrebs, Schilddrüsenkrebs, Blasenkrebs, bösartiger Hauttumor oder Lymphom oder Leukämie ist.

2. Isovalerylspiramycin I oder III zur Verwendung gemäß Anspruch 1, wobei der Hirntumor ein Gliom oder Meningeom ist und der Magenkrebs gastrisches Adenokarzinom ist.

3. Isovalerylspiramycin I oder III zur Verwendung gemäß Anspruch 1 oder 2, wobei ein Medikament in verschiedenen Formulierungen vorliegt, die Isovalerylspiramycin I oder III und pharmazeutisch akzeptable Adjuvantien umfassen, oder
das Medikament in verschiedenen Formulierungen vorliegt, die pharmazeutisch akzeptable Salze von Isovalerylspiramycin I oder III und pharmazeutisch akzeptable Adjuvantien umfassen.

4. Isovalerylspiramycin I oder III zur Verwendung gemäß Anspruch 1 oder 2, wobei ein Medikament in verschiedenen Formulierungen vorliegt, die Isovalerylspiramycin I oder III, Anti-Tumor-Arzneimittel und die pharmazeutisch akzeptablen Adjuvantien umfassen, oder
das Medikament in verschiedenen Formulierungen vorliegt, die pharmazeutisch akzeptable Salze von Isovalerylspiramycin I oder III, Anti-Tumor-Arzneimittel und die pharmazeutisch akzeptablen Adjuvantien umfassen.

5. Isovalerylspiramycin I oder III zur Verwendung gemäß Anspruch 1 oder 2, wobei ein Medikament eine Kombination aus einem ersten Wirkstoff und einem zweiten Wirkstoff ist, der erste Wirkstoff Isovalerylspiramycin I oder III und die pharmazeutisch akzeptablen Salze von Isovalerylspiramycin I oder III enthält und der zweite Wirkstoff ein Anti-Tumor-Arzneimittel enthält.

6. Isovalerylspiramycin I oder III zur Verwendung gemäß Anspruch 4 oder 5, wobei das Anti-Tumor-Arzneimittel ein oder mehr als ein Arzneimittel ist, ausgewählt aus einer Gruppe, enthaltend ein Chemotherapie-Arzneimittel, ein Strahlentherapie-Arzneimittel, ein Arzneimittel für gezielte Therapie und ein immuntherapeutisches Arzneimittel enthält.

7. Isovalerylspiramycin I oder III zur Verwendung gemäß Anspruch 4 oder 5, wobei eine Dosis von Isovalerylspiramycin I oder III in dem Medikament in einem Bereich von 5 bis 1500 mg liegt.

8. Isovalerylspiramycin I oder III zur Verwendung gemäß Anspruch 7, wobei eine Dosis von Isovalerylspiramycin I oder III in dem Medikament in einem Bereich von 50 bis 1000 mg liegt.

9. Isovalerylspiramycin I oder III zur Verwendung gemäß Anspruch 8, wobei eine Dosis von Isovalerylspiramycin I oder III in dem Medikament in einem Bereich von 100 bis 400 mg liegt.

## Revendications

1. Isovaléryl spiramycine I ou III pour son utilisation dans le traitement et/ou la prévention des tumeurs, où la tumeur est un cancer du sein, un cancer du foie, un cancer du poumon, un cancer du rein, une tumeur du cerveau, un cancer du col de l'utérus, un cancer de la prostate, un lymphome, un cancer du pancréas, un cancer de l'oesophage, un cancer de l'estomac, un cancer du côlon, un cancer de la thyroïde, un cancer de la vessie, une tumeur maligne de la peau, ou un lymphome ou une leucémie.

2. Isovaléryl spiramycine I ou III pour son utilisation selon la revendication 1, où la tumeur du cerveau est un gliome ou un méningiome, et le cancer de l'estomac est un adénocarcinome de l'estomac.

3. Isovaléryl spiramycine I ou III pour son utilisation selon la revendication 1 ou la revendication 2, où un médicament est de formulations diverses incluant de l'isovaléryl spiramycine I ou III et des adjuvants pharmaceutiquement acceptables, ou
le médicament est de formulations diverses incluant des sels pharmaceutiquement acceptables d'isovaléryl spiramycine I ou III et des adjuvants pharmaceutiquement acceptables.

4. Isovaléryl spiramycine I ou III pour son utilisation selon la revendication 1 ou la revendication 2, où un médicament est de formulations diverses incluant de l'isovaléryl spiramycine I ou III, des médicaments antitumoraux et les adjuvants pharmaceutiquement acceptables, ou
le médicament est de formulations diverses incluant les sels pharmaceutiquement acceptables d'isovaléryl spiramycine I ou III, des médicaments antitumoraux et les adjuvants pharmaceutiquement acceptables.

5. Isovaléryl spiramycine I ou III pour son utilisation selon la revendication 1 ou la revendication 2, où un médicament est une combinaison d'un premier agent et d'un deuxième agent, le premier agent contient de l'isovaléryl spiramycine I ou III et les sels pharmaceutiquement acceptables d'isovaléryl spiramycine I ou III, et le deuxième agent contient un médicament antitumoral.

6. Isovaléryl spiramycine I ou III pour son utilisation selon la revendication 4 ou la revendication 5, où le médicament antitumoral est un médicament ou plus d'un médicament sélectionnés dans un groupe contenant un médicament de chimiothérapie, un médicament de radiothérapie, un médicament de thérapie ciblée, et un médicament immunothérapeutique.

7. Isovaléryl spiramycine I ou III pour son utilisation selon la revendication 4 ou la revendication 5, où une dose d'isovaléryl spiramycine I ou III dans le médicament est comprise dans un intervalle allant de 5 à 1 500 mg.

8. Isovaléryl spiramycine I ou III pour son utilisation selon la revendication 7, où une dose d'isovaléryl spiramycine I ou III dans le médicament est comprise dans un intervalle allant de 50 à 1 000 mg.

9. Isovaléryl spiramycine I ou III pour son utilisation selon la revendication 8, où une dose d'isovaléryl spiramycine I ou III dans le médicament est comprise dans un intervalle allant de 100 à 400 mg.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3607952 A1
- WO 2007144876 A1
- EP 2578596 A1
- CN 101785778 A
- CN 101785779 A
- CN 101773510 A